# EUROPEAN PATENT SPECIFICATION

(11) **EP 2 007 738 B1**
(45) Date of publication and mention of the grant of the patent: **26.02.2014**
(21) Application number: 07723743.6
(22) Date of filing: 29.03.2007
(51) Int. Cl.: C07D 251/18, C07D 407/12, A01N 43/68

(54) **DIAMINO-1 ,3,5-TRIAZINES N-SUBSTITUTED WITH BICYCLIC RADICALS, PROCESS FOR THEIR PREPARATION, COMPOSITIONS THEREOF, AND THEIR USE AS HERBICIDES AND PLANT GROWTH REGULATORS**
MIT BICYCLISCHEN RADIKALEN N-SUBSTITUIERTE DIAMINO-1 ,3,5-TRIAZINE, VERFAHREN ZU IHRER HERSTELLUNG, ZUSAMMENSETZUNGEN DARAUS UND IHRE VERWENDUNG ALS HERBIZIDE UND PFLANZENWACHSTUMSREGULATOREN
DIAMINO-1,3,5-TRIAZINES N-SUBSTITUEES AVEC DES RADICAUX BICYCLIQUES, PROCEDE DE PREPARATION DE CEUX-CI, COMPOSITIONS LES CONTENANT ET UTILISATION DE CEUX-CI EN TANT QU'HERBICIDES ET REGULATEURS DE CROISSANCE DE PLANTES

(30) Priority: 11.04.2006 DE 102006016885
(43) Date of publication of application: 31.12.2008
(73) Proprietor: Bayer CropScience AG, 40789 Monheim (DE)
(72) Inventor: MINN, Kiemens, 65795 Hattersheim (DE); DIETRICH, Hansjörg, 65719 Hofheim (DE); HILLS, Martin, 65510 Idstein (DE); KEHNE, Heinz, 65719 Hofheim (DE); ROSINGER, Christopher, 65719 Hofheim (DE); FEUCHT, Dieter, 65760 Eschborn (DE)
(86) International application number: PCT/EP2007/002799
(87) International publication number: WO 2007/115695

(56) References cited:
- EP-A1- 0 864 567
- WO-A-97/31904
- WO-A-2004/069814

## Description

The invention relates to the field of agrochemicals, particularly agrochemicals for the control of weeds or undesired vegetation and for plant protection.

### Background of the invention

WO-A-97/31904 describes the preparation of 2-amino-4-bicyclyl-amino-1,3,5-triazines and their use as herbicides and plant growth regulators. The reference generically describes various amino-1,3,5-triazine derivatives which are substituted with some radicals comprising one or more centers of chirality in principle. WO-A-2004/069814 describes specific optical isomers of 2-amino-4-bicyclyl-amino-1,3,5-triazines. Some of the specifically described active substances do not have optimal applicational properties, i. e. they have disadvantages when used, for example have an insufficient herbicidal action against harmful plants, too rigid applicational limitations related to weather, climate and/or soil conditions, too narrow a spectrum against weeds or too little crop selectivity.

It has now been found that surprisingly specific 2,4-diamino-1,3,5-triazines, bearing substituted bicyclic radicals at one amino group and specific radicals at the triazine ring possess advantageous applicational properties compared with known structurally close compounds. For instance, they are very active herbicides which can be used for the control of a range of harmful weeds, having surprising potent herbicidal efficacy or scope of weed spectrum to be controlled.

### Detailed Description of the Invention

The present invention relates to compounds of formula (I) or salts thereof, 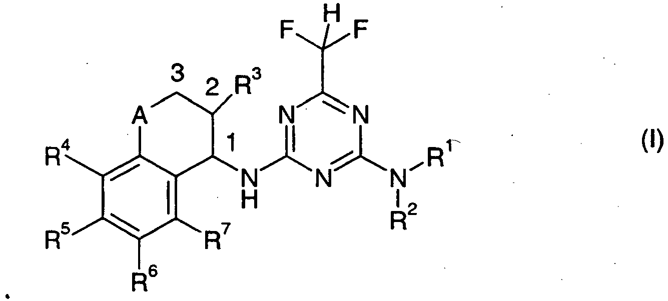 in which:
- R¹ and R²: are each independently H, (C₁-C₄)alkyl, (C₁-C₄)haloalkyl, (C₃-C₄)alkenyl, (C₃-C₄)haloalkenyl, (C₃-C₄)alkynyl, (C₃-C₄)haloalkynyl or an acyl radical,
- R³: is H, (C₁-C₆)alkyl, (C₁-C₆)alkoxy, (C₂-C₆)alkenyl, (C₂-C₆)alkinyl, (C₂-C₆)alkenyloxy or (C₂-C₆)alkinyloxy, preferably H, (C₁-C₆)alkyl or (C₁-C₆)alkoxy,
- R⁴, R⁵, R⁶ and R⁷: are each independently H, (C₁-C₄)alkyl, (C₁-C₃)haloalkyl, halogen, (C₁-C₃)alkoxy, (C₁-C₃)haloalkoxy, CN, (C₂-C₆)alkenyl, (C₂-C₆)alkinyl, (C₂-C₆)alkenyloxy or (C₂-C₆)alkinyloxy, but at least one of them is (C₁-C₄)alkyl, (C₁-C₃)haloalkyl, halogen, (C₁-C₃)alkoxy, (C₁-C₃)haloalkoxy, CN, (C₂-C₆)alkenyl, (C₂-C₆)alkinyl, (C₂-C₆)alkenyloxy or (C₂-C₆)alkinyloxy, preferably
- R⁴, R⁵, R⁶ and R⁷: are each independently H, (C₁-C₄)alkyl, (C₁-C₃)haloalkyl, halogen, (C₁-C₃)alkoxy, (C₁-C₃)haloalkoxy or CN, but at least one of them is (C₁-C₄)alkyl, (C₁-C₃)haloalkyl, halogen, (C₁-C₃)alkoxy, (C₁-C₃)haloalkoxy or CN,
- A: is CH₂, O or a direct bond.

For reference purposes in formula (I) certain ring carbon atoms are marked from 1 to 3. In the chemical names for the compounds of formula (I) the substitutents R⁴, R⁵, R⁶ and R⁷ are numbered consecutively according to IUPAC nomenclature wherein the numbering starts with the saturated ring beginning with the carbon atom attached to the nitrogen of the amino group and anti-clockwise to the aromatic ring while leaving the C-atoms of the bridge without number. For instance if A is a CH₂ group R⁴ is in position 5, R⁵ in position 6, R⁶ in position 7 and R⁷ in position 8; if A is a direct bond R⁴ is in position 4, R⁵ in position 5, R⁶ in position 6 and R⁷ in position 7. If A is a heteroatom the numbering of the position R⁴, R⁵, R⁶ and R⁷ depends on whether the heteroatom position is numbered 1 or the carbon atom attached to the amino group is numbered 1.

The compounds of the formula (I) can form salts by addition of a suitable inorganic or organic acid such as, for example, HCl, HBr, H₂SO₄ or HNO₃, or a mono- or bifunctional carboxylic acid or sulfonic acid, to a basic group such as, for example, amino or alkylamino.

In the present patent specification, including the accompanying claims, the aforementioned substituents have the following meanings:

Halogen means fluorine, chlorine, bromine or iodine.

The term "halo" before the name of a radical means that this radical is partially or completely halogenated, that is to say substituted by F, Cl, Br or I in any combination.

The expression "(C₁-C₆)alkyl" means an unbranched or branched non-cyclic saturated hydrocarbon radical having 1, 2, 3, 4, 5 or 6 carbon atoms (indicated by a range of C-atoms in the parenthesis), such as, for example a methyl, ethyl, propyl, isopropyl, 1-butyl, 2-butyl, 2-methylpropyl or tert-butyl radical. The same applies to alkyl groups in composite radicals such as "alkoxyalkyl".

Alkyl radicals and also in composite groups, unless otherwise defined, preferably have 1 to 4 carbon atoms.

"(C₁-C₆)Haloalkyl" means an alkyl group mentioned under the expression "(C₁-C₆)alkyl" in which one or more hydrogen atoms are replaced by the same number of identical or different halogen atoms, such as monohaloalkyl, perhaloalkyl, CF₃, CHF₂, CH₂F, CHFCH₃, CF₃CH₂, CF₃CF₂, CHF₂CF₂, CH₂FCHCl, CH₂Cl, CCl₃, CHCl₂ or CH₂CH₂Cl.

"[(C₁-C₄)alkoxy](C₁-C₆)alkyl" means (C₁ -C₆)alkyl which is substituted by (C₁-C₄)alkoxy.

"(C₁-C₆)Alkoxy" means an alkoxy group whose carbon chain has the meaning given under the expression "(C₁-C₆)alkyl". "Haloalkoxy' is, for example, OCF₃, OCHF₂, OCH₂F, CF₃CF₂O, OCH₂CF₃ or OCH₂CH₂Cl.

"(C₂-C₆)Alkenyl" means an unbranched or branched non-cyclic carbon chain having a number of carbon atoms which corresponds to this stated range and which contains at least one double bond which can be located in any position of the respective unsaturated radical. "(C₂-C₆)alkenyl" accordingly denotes, for example, the vinyl, allyl, 2-methyl-2-propenyl, 2-butenyl, pentenyl, 2-methylpentenyl or the hexenyl group.

"(C₂-C₆)alkynyl" means an unbranched or branched non-cyclic carbon chain having a number of carbon atoms which corresponds to this stated range and which contains one triple bond which can be located in any position of the respective unsaturated radical. "(C₂-C₆)alkynyl" accordingly denotes, for example, the propargyl, 1-methyl-2-propynyl, 2-butynyl or 3-butynyl group.

"(C₃-C₆)cycloalkyl" denotes monocyclic alkyl radicals, such as the cyclopropyl, cyclobutyl, cyclopentyl or cyclohexyl radical.

"(C₄-C₆)cycloalkenyl" denotes a carbocyclic, nonaromatic, partially unsaturated ring having 4 to 6 carbon atoms, for example 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl.

An acyl radical is, in a broad sense, the radical of an organic acid which is formed formally by removing an OH group, for example the radical of a carboxylic acid and radicals of acids derived therefrom, such as thiocarboxylic acid, unsubstituted or N-substituted iminocarboxylic acids or the radical of carbonic monoesters, unsubstituted or N-substituted carbamic acid, unsubstituted or N-substituted thiocarbamic acid, sulfonic acids, sulfinic acids, phosphonic acids, and phosphinic acids. Acyl is, for example, formyl, alkylcarbonyl such as [(C₁-C₄)alkyl]carbonyl, phenylcarbonyl, alkyloxycarbonyl, phenyloxycarbonyl, benzyloxycarbonyl, alkylsulfonyl, alkylsulfinyl, phenylsulfonyl, N-alkyl-1-iminoalkyl and other radicals of organic acids. In this context, the radicals can be even further substituted in each of the alkyl or phenyl moieties, for example in the alkyl moiety by one or more radicals selected from the group consisting of halogen, alkoxy, phenyl and phenoxy; examples of substituents in the phenyl moiety are mono- or polysubstituted, preferably up to trisubstituted, identical or different radicals selected from the group consisting of halogen, (C₁-C₄)alkyl, (C₁-C₄)alkoxy, (C₁-C₄)haloalkyl, (C₁-C₄)haloalkoxy and nitro, for example o-, m- and p-tolyl, dimethylphenyls, 2-, 3- and 4-chlorophenyl, 2-, 3- and 4-trifluoro- and -trichlorophenyl, 2,4-, 3,5-, 2,5- and 2,3-dichlorophenyl, o-, m- and p-methoxyphenyl.

The acyl radical generally has 1 to 24 carbon atoms, preferably 1 to 18, more preferably 1 to 12, most preferably 1 to 7, in particular 1 to 4.

Acyl in the narrower sense is, for example, the radical of an alkanoic acid, alkenoic acid, alkynoic acid, arylcarboxylic acid (for example benzoyl), alkoxycarbonyl, alkenyloxycarbonyl, alkynyloxycarbonyl, aryloxycarbonyl, alkylsulfonyl, alkysulfinyl or phenylsulfonyl; in an even narrower sense, acyl is a radical of an alkanoic acid, for example a (C₁-C₂₄)alkanoic acid, preferably (C₁-C₁₈)alkanoic acid, in particular (C₁-C₁₂)alkanoic acid, very especially (C₁-C₆)alkanoic acid such as formyl, acetyl or propionyl.

The expression "one or more radicals selected from the group consisting of" in the definition is to be understood as meaning in each case one or more identical or different radicals selected from the stated group of radicals, unless specific limitations are defined expressly.

By combination of variables the generic formulae may formally define unstable functional groups, e.g. the carbamyl radical or the hydroxy carbonyloxy radical, which are unstable in neutral or acidic aqueous medium and which thus are not preferred or are used by way of their stable salts or degradation products only, respectively.

Compounds of the stated formula (I) according to the invention or their salts in which individual radicals have one of the preferred meanings which have already been stated or are stated hereinbelow and particularly those shown in the Table examples, or in particular those in which two or more of the preferred meanings which have already been stated or which are stated hereinbelow are combined, are of particular interest, mainly because of the more potent herbicidal action, better selectivity and/or greater ease of preparation.

Of particular interest are compounds of formula (I) where a radical selected from the group of radicals R¹, R², R³, R⁴, R⁵, R⁶, R⁷and A is preferably defined as set forth below, wherein the definition of the radical is independent from the definitions of the other radicals of said group. Preferred compounds of formula (I) contain a combination of radicals of said group which comprise two or more preferred meanings set forth below.

In the following preferred definitions it is generally to be understood that where symbols are not specifically defined for a specific group of compounds they are to be defined as defined for the compounds in formula (I) or the respective generic formula or for preferred compounds of formula (I) or the respective preferred formula in the description.

Preferably R¹ is H, (C₁-C₄)alkyl, (C₁-C₄)haloalkyl, (C₃-C₄)alkenyl, (C₃-Ca)alkynyl or an acyl radical having 1 to 12 carbon atoms.

An acyl radical is herewith preferably CHO, -CO(C₁-C₆)alkyl, -CO(C₁-C₆)haloalkyl, -CO₂(C₁-C₆)alkyl, -SO₂(C₁-C₆)alkyl, -CO₂-phenyl or -CO-phenyl wherein each phenyl is unsubstituted or substituted by one or more radicals selected from the group consisting of halogen, (C₁-C₂)alkyl, (C₁-C₂)haloalkyl, (C₁-C₂)alkoxy, (C₁-C₂)haloalkoxy and NO₂.

More preferably R¹ is H, (C₁-C₃)alkyl, (C₁-C₃)haloalkyl, allyl, propargyl, CHO, -CO(C₁-C₃)alkyl or -CO(C₁-C₃)haloalkyl; yet more preferably R¹ is H, CHO, COCH₃, COCH₂Cl, COCH(CH₃)Cl or COCF₃; most preferably R¹ is H.

Preferably R² is H, (C₁-C₄)alkyl or (C₁-C₄)haloalkyl; more preferably R² is H or (C₁-C₂)alkyl; most preferably R² is H.

Preferably R³ is H, (C₁-C₃)alkyl, (C₂-C₄)alkenyl, (C₂-C₄)alkinyl, (C₁-C₃)alkoxy, (C₂-C₄)alkenyloxy or (C₂-C₄)alkinyloxy, more preferably R³ is H, (C₁-C₃)alkyl or (C₁-C₃)alkoxy, more preferably R³ is H or (C₁-C₃)alkyl, more preferably R³ is H, methyl or ethyl.

Preferably R⁴, R⁵, R⁶ and R⁷ are each independently H, (C₁-C₄)alkyl, (C₁-C₃)haloalkyl, halogen, (C₁-C₃)alkoxy, (C₁-C₃)haloalkoxy, CN, (C₃-C₄)alkenyl, (C₃-C₄)alkinyl, (C₃-C₄)alkenyloxy or (C₃-C₄)alkinyloxy, but at least one of them is (C₁-C₄)alkyl, (C₁-C₃)haloalkyl, halogen, (C₁-C₃)alkoxy, (C₁-C₃)haloalkoxy, CN, (C₃-C₄)alkenyl, (C₃-C₄)alkinyl, (C₃-C₄)alkenyloxy or (C₃-C₄)alkinyloxy; more preferably

R⁴, R^{S}, R⁶ and R⁷ are each independently H, (C₁-C₄)alkyl, (C₁-C₃)haloalkyl, halogen, (C₁-C₃)alkoxy, (C₁-C₃)haloalkoxy or CN, but at least one of them is (C₁-C₄)alkyl, (C₁-C₃)haloalkyl, halogen, (C₁-C₃)alkoxy, (C₁-C₃)haloalkoxy or CN.

More preferably R⁴, R⁵, R⁶ and R⁷ are each independently H, (C₁-C₄)alkyl, halogen or (C₁-C₃)alkoxy, but at least one of these, preferably one of these is (C₁-C₄)alkyl, (C₁-C₃)haloalkyl, halogen or (C₁-C₃)alkoxy.

More preferably R⁴, R⁵, R⁶ and R⁷ are each independently H, methyl, F, Cl or Br but at least one of these, preferably one of these is methyl, F, Cl or Br.

More preferably R⁴, R⁵ and R⁷ are each independently H, (C₁-C₃)alkyl, halogen or (C₁-C₃)alkoxy and R⁶ is (C₁-C₃)alkyl, halogen or (C₁-C₃)alkoxy; more preferably R⁴, R⁵and R⁷ are each independently H, methyl, F or Cl and R⁶ is methyl, F, Cl or Br.

Preferably A is CH₂ or a direct bond, particularly a direct bond.

Preferred compounds of formula (I) are those in which
R¹ is H, (C₁-C₄)alkyl, (C₁-C₄)haloalkyl, (C₃-C₄)alkenyl, (C₃-C₄)alkynyl or an acyl radical having 1 to 12 carbon atoms, wherein acyl preferably is CHO, -CO(C₁-C₆)alkyl, -CO(C₁-C₆)haloalkyl, -CO₂(C₁-C₆)alkyl, -SO₂(C₁-C₆)alkyl, -CO₂-phenyl or -CO-phenyl wherein each phenyl is unsubstituted or substituted by one or more radicals selected from the group consisting of halogen, (C₁-C₂)alkyl, (C₁-C₂)haloalkyl, (C₁-C₂)alkoxy, (C₁-C₂)haloalkoxy and NO₂,
R² is H, (C₁-C₄)alkyl or (C₁-C₄)haloalkyl, preferably H or (C₁-C₂)alkyl, more preferably H,
R³ is H, (C₁-C₃)alkyl or (C₁-C₃)alkoxy, preferably H or (C₁-C₃)alkyl, more preferably H, methyl or ethyl;
R⁴, R⁵, R⁶ and R⁷ are each independently H, (C₁-C₃)alkyl, halogen or (C₁-C₃)alkoxy, but at least one of them is (C₁-C₃)alkyl, halogen or (C₁-C₃)alkoxy, and A is CH₂, O or a direct bond, preferably A is CH₂ or a direct bond, in particular a direct bond.

More preferred compounds of formula (I) are those in which:
R¹ is H, (C₁-C₃)alkyl, (C₁-C₃)haloalkyl, allyl, propargyl, CHO, -CO(C₁-C₃)alkyl or -CO(C₁-C₃)haloalkyl, preferably H, CHO, COCH₃, COCH₂Cl, COCH(CH₃)Cl or COCF₃, more preferably H,
R² is H or (C₁-C₂)alkyl, preferably H,
R³ is H, (C₁-C₃)alkyl or (C₁-C₃)alkoxy, preferably H or (C₁-C₃)alkyl, more preferably H, methyl or ethyl,
R⁴, R⁵, R⁶ and R⁷ are each independently H, methyl, F, Cl or Br, preferably H, methyl, F or Cl, but at least one of them is methyl, F, Cl or Br, preferably methyl, F or Cl, and
A is CH₂, 0 or a direct bond, preferably A is CH₂ or a direct bond, in particular a direct bond.

Also preferred compounds of formula (I) are those in which:
R¹ is H, (C₁-C₄)alkyl, (C₁-C₄)haloalkyl, (C₃-C₄)alkenyl, (C₃-C₄)alkynyl or an acyl radical having 1 to 12 carbon atoms, wherein acyl preferably is CHO, -CO(C₁-C₆)alkyl, -CO(C₁-C₆)haloalkyl, -CO₂(C₁-C₆)alkyl, -SO₂(C₁-C₆)alkyl, -CO₂-phenyl or -CO-phenyl wherein each phenyl is unsubstituted or substituted by one or more radicals selected from the group consisting of halogen, (C₁-C₂)alkyl, (C₁-C₂)haloalkyl, (C₁-C₂)alkoxy, (C₁-C₂)haloalkoxy and NO₂,
R² is H, (C₁-C₄)alkyl or (C₁-C₄)haloalkyl, preferably H or (C₁-C₂)alkyl, more preferably H,
R³ is H, (C₁-C₃)alkyl or (C₁-C₃)alkoxy, preferably H or (C₁-C₃)alkyl, more preferably H, methyl or ethyl,
R⁴, R⁵ and R⁷ are each independently H, (C₁-C₃)alkyl, halogen or (C₁-C₃)alkoxy, and R⁶ is (C₁-C₃)alkyl, halogen or (C₁-C₃)alkoxy, and
A is CH₂, O or a direct bond, wherein preferably A is CH₂ or a direct bond, in particular a direct bond.

More preferred compounds of formula (I) are those in which:
R¹ is H, (C₁-C₃)alkyl, (C₁-C₃)haloalkyl, allyl, propargyl, CHO, -CO(C₁-C₃)alkyl or -CO(C₁-C₃)haloalkyl, preferably H, CHO, COCH₃. COCH₂Cl, COCH(CH₃)Cl or COCF₃, more preferably H,
R² is H or (C₁-C₂)alkyl, preferably H,
R³ is H, (C₁-C₃)alkyl or (C₁-C₃)alkoxy, preferably H or (C₁-C₃)alkyl, more preferably H, methyl or ethyl,
R⁴, R⁵ and R⁷ are each independently H, methyl, F, Cl or Br, preferably H, methyl, F or Cl,
R⁶ is methyl, F, Cl or Br, preferably methyl, F or Cl, and
A is CH₂, O or a direct bond, wherein preferably A is CH₂ or a direct bond, in particular a direct bond.

Especially preferred compounds of formula (I) are those in which:
R¹ is H,
R² is H,
R³ is H or (C₁-C₃)alkyl, preferably H, methyl or ethyl,
R⁴, R⁵ and R⁷ are each independently selected from the group consisting of H, methyl, F, Cl and Br, preferably H, methyl, F and Cl and R⁶ is methyl, F, Cl or Br, preferably is methyl, F or Cl, and
A is CH₂, O or a direct bond, preferably CH₂ or a direct bond, in particular a direct bond.

The compounds of formula (I) contain at least one center of asymmetry at the marked 1 position and comprise all stereoisomers which are encompassed by formula (I) with respect to marked 1 position and optionally additional centers of asymmetry, and their mixtures. The last-mentioned compounds of the formula (I) can contain one or more additional asymmetric carbon atoms and/or double bonds which are not stated specifically in the formula (I). It will be understood that the present invention embraces both the pure isomers and mixtures thereof, including the racemic mixtures.

The possible stereoisomers which are defined by their specific spatial form, such as enantiomers, diastereomers, Z- and E-isomers, are all encompassed by formula (I) and can be obtained by customary methods from mixtures of the stereoisomers, or else be prepared by stereoselective reactions in combination with the use of stereochemically pure starting materials.

The invention also encompasses any keto and enol tautomer forms and mixtures and salts thereof, if respective functional groups are present.

With respect to the center of asymmetry at the marked 1 position the stereoisomers were found to differ in their properties, such as in the herbicidal efficacy. Preferred compounds of formula (I) are compounds of the formula (Ia) or salts thereof, 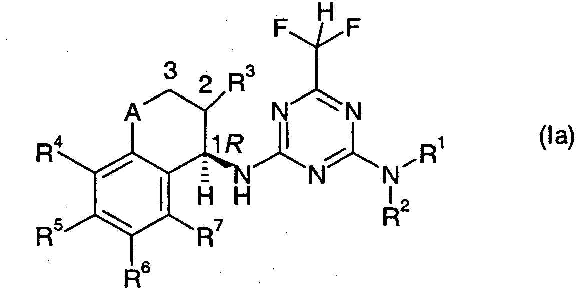 in which the stereochemical configuration at the marked 1 position is 60 to 100 % (R), preferably 70-100 % (R), in particular 80-100 % (R), based on the content of stereoisomers having (R)- and (S)-configurations at this position.

The stereochemical configuration at the marked 1 position of the preferred compounds (Ia) is predominantly (R) according to the Cahn-Ingold-Prelog system, provided that the 4 substituents at the chiral C-atom marked 1 having the following ranking:
1. N-atom,
2. next C-atom in the benzene ring,
3. ring C-atom of group CHR³,
4. hydrogen atom,
(see also orientation of bonds indicated in formula (Ia) for the "(*R*)-isomer"). While the stereochemical configuration of compounds (Ia) is herewith generally designated as (*R*)-configuration it has to be noted that specific compounds of formula (Ia) having same stereochemical structure correspond to the (*S*)-configuration according to the nomenclature of Cahn-Ingold-Prelog. For instance if R*³* is an alkoxy group, the group CHR³ normally would have a rank before the next C-atom of the benzene ring. Therefore the configuration of those alkoxysubstituted compounds of formula (Ia) normally is the (*S*)-configuration according to the stereochemical nomenclature of Cahn-Ingold-Prelog. However, in the context of the general formula (Ia) and other general formulae of optically active compounds mentioned below those compounds are compounds representing the "*(*R)-configuration" with respect to the C-atom marked 1, applying the general ranking of substituents as mentioned for formula (Ia) above. If a full chemical name of a specific compound of formula (Ia) or of other formulae of optically active compounds is given, however, the nomenclature of Cahn-Ingold-Prelog is strictly applied unless mentioned otherwise, and such a compound of formula (Ia) can then have the (*S*)-configuration as explained above.

The preferred compounds (Ia) also comprise all stereoisomers which are encompassed by formula (I) due to additional centers of asymmetry, and their mixtures. Such compounds of the formula (I) contain, e. g. one or more additional asymmetric carbon atoms or else double bonds which are not stated specifically in the formula (I). It will be understood that the present invention embraces both the pure isomers and more or less enriched mixtures thereof, where the asymmetric carbon atom in marked 1 position is in the (*R*)-configuration or, in mixtures, a compound or compounds of same chemical constitution have the (*R*)-configuration in marked 1 position or are present in a ratio that compounds having the *R-*configuration are predominantly present (at least 60 % (*R*)-configuration) whilst the other asymmetric carbon atom(s) may be present in racemic form or are more or less resolved too. Provided the condition for the stereochemical configuration at marked 1 position is met, the possible stereoisomers which are defined by their specific spatial form, such as enantiomers, diastereomers, Z- and E-isomers, are all encompassed by formula (I) and can be obtained by customary methods from mixtures of the stereoisomers, or else be prepared by stereoselective reactions in combination with the use of stereochemically pure starting materials.

A further centre of asymmetry may be present at the carbon atom marked 2 in formula (I), provided that the R³ group is different from hydrogen, in which case the compound of the invention can exist as at least two pure stereoisomeric forms, the additional asymmetric carbon atom having (*R*) or (*S*) configuration according to the Cahn-Ingold-Prelog system, i.e. pure isomers having the configuration (1*R*, 2*R*) or (1*R*, 2*S*) selected from the four pure stereoisomers which exist in principle. Moreover depending on the nature of the groups R¹, R², R⁴, R⁵, R⁶ and R⁷ further asymmetric carbon atoms may be present.

Preferred are also compounds of formulae (Ib) and (Ic), which are stereoisomers of the compounds of general formula (I) or (Ia) wherein the configurations at the carbon atoms marked 1 and 2 are specifically defined, 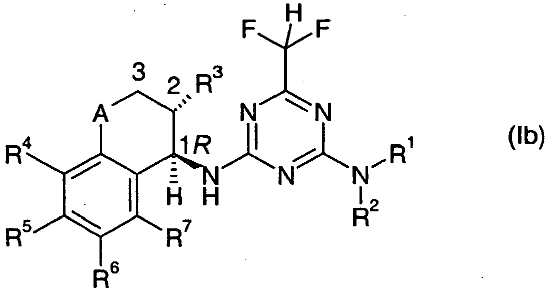 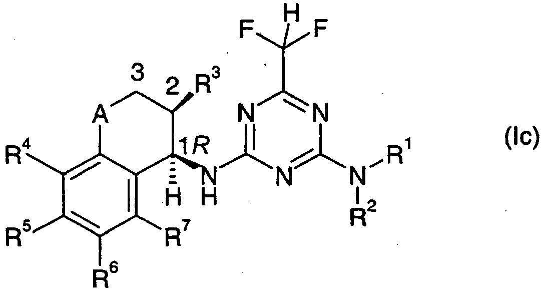 in which R¹, R², R³, R⁴, R⁵, R⁶, R⁷and A are as defined in formula (I), provided that the R³ group is different from hydrogen, and in which the stereochemical configuration at the marked 1 position is 100 % (*R*), or also the optically active substance mixtures of enantiomers having 60 to 100 % (*R*), preferably 70-100 % (*R*), in particular 80-100 % (*R*), based on the content of stereoisomers having (*R*)-and (*S*)-configurations at this position. The definition of the (*R*)-configuration is thereby defined by the formula shown and in compliance with the definition mentioned for formula (Ia) above already.

In the compounds (Ib) the group R³ is oriented in trans-position relative to the vicinal N-atom.

In the compounds (Ic) the group R³ is cis-oriented relative to the vicinal N-atom.

Preferred are compounds of formula (I), (Ia), (Ib) or (Ic) in which
R³ is (C₁-C₆)alkyl or (C₁-C₄)alkoxy; and
the other symbols are as defined in formula (I) or as preferably defined for formula (I).

More preferred are compounds of formula (Ib) and (Ic), wherein
R¹ and R² are each H,
R³ is methyl or ethyl,
R⁴, R⁵, R⁶ and R⁷ are each independently H, methyl, Br, Cl or F but at least one of them is methyl, Br, CI or F, and
A is CH₂, O or a direct bond.

Also more preferred are compounds of formula (Ia) and (Ib), wherein
R¹ and R² are each H, and
R³ is methyl or ethyl,
R⁴, R⁵ and R⁷ are each independently H, methyl, Br, Cl or F, and
R⁶ is methyl, Br, Cl or F, and
A is CH₂, O or a direct bond.

Preferred are also the compounds (Ib) or (Ic) in mixtures of stereoisomers where the stereochemical configuration at the marked 1 position is 60 to 100 % (*R*), preferably 70 to100 % (*R*), in particular 80 to100 % (*R*), based on the content of stereoisomers having (*R*)- and (*S*)-configurations at marked 1 position. For example, mixtures of compounds (Ib) and (Ic), such as the 50:50 mixture having the configuration (1*R*,2*RS*) are also preferred as well as mixtures having a ratio where (Ib) or (Ic) is present in an excess.

Also preferred are the racemic compounds wherein the compounds (Ib) (1,2-trans compound) or (Ic) (1,2-cis compound) are present together with their (*1S*)-enantiomers.

Compounds of formula (I) above may be prepared by the application or adaptation of known methods (i.e. methods heretofore used or described in the literature), for example as generally described in WO-A-97/29095, WO-A-2004/069814 and references cited therein, and as hereinafter described.

In the following description where symbols appearing in formulae are not specifically defined, it is to be understood that they are "as hereinbefore defined" in accordance with the first definition of each symbol in the specification or the respective preferred definitions.

It is to be understood that in the descriptions of the following processes the sequences may be performed in different orders, and that suitable protecting groups may be required to achieve the compounds sought.

According to a feature of the present invention compounds of formula (I) can be prepared by the reaction of a compound of general formula (II), 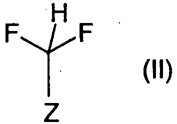 wherein Z is a functional group selected from the group consisting of carboxylic ester, carboxylic orthoester, carboxylic acid chloride, carboxamide, cyano, carboxylic anhydride or trichloromethyl, with a biguanidine compound of formula (III) or an acid addition salt thereof, 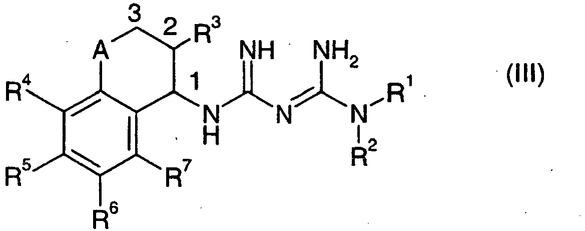 wherein R¹, R², R³, R⁴, R⁵, R⁶, R⁷ and A and the configuration at the marked 1 position are as defined in the compound of formula (I) to be prepared.

For the prepation of compounds of formula (Ia) preferably compounds of formula (IIIa) are used in the process, 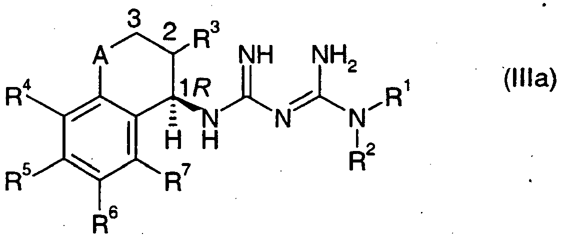 in which R¹, R², R³, R⁴, R⁵, R⁶, R⁷ and A and the configuration at the marked 1 position are as defined in the compound of formula (Ia) to be prepared.

The reaction generally can be performed, e. g. in the presence of a base, in an inert solvent such as tetrahydrofuran, dioxan, acetonitrile, N,N-dimethylformamide, methanol or ethanol, at a temperature of from 0°C to the reflux temperature of the solvent, preferably at 20°C to 60°C. For instance, the base can be an alkali metal hydroxide, alkali metal hydride, alkali metal carbonate, alkali metal alkoxide, alkaline earth metal carbonate, or an organic base such as a tertiary amine for example triethylamine, or 1,8-diazabicyclo[5.4.0]undec-7-en (DBU).

According to a further feature of the present invention said compounds of formula (I), or preferably (Ia), may also be prepared by the reaction of a compound of general formula (IV), 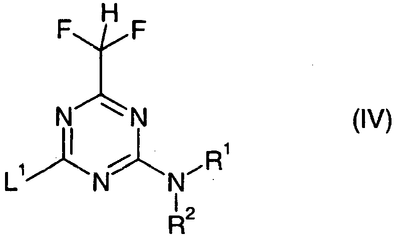 in which R¹ and R² are as defined in the compound of formula (I) to be prepared, and L¹ is a leaving group such as chlorine, trichloromethyl, (C₁-C₄)-alkylsulfonyl, phenylsulfonyl or (C₁-C₄)alkyl-phenylsulfonyl, with an amine of formula (V) or (Va), respectively, or an acid addition salt thereof, 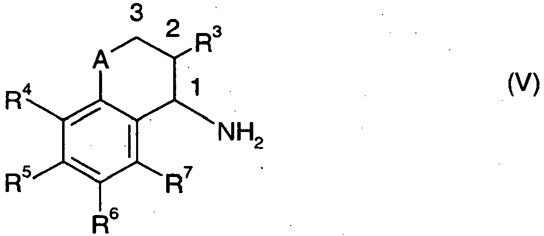 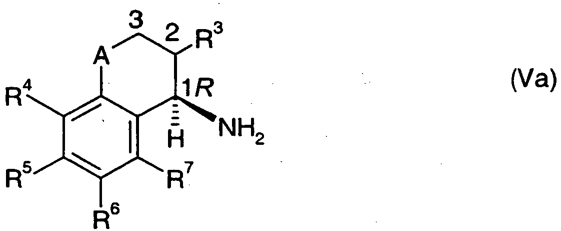 in which R³, R⁴, R⁵, R⁶, R⁷ and A are as defined in the compound of formula (I) to be prepared.

The reaction is generally performed in the presence of a base, in an inert solvent, e. g. a polar organic solvent such as tetrahydrofuran, dioxan, acetonitrile, N,N-dimethylformamide, methanol or ethanol, at a temperature of from 0°C to the reflux temperature of the solvent, preferably at 20°C to 100°C. The base is generally an alkali metal hydroxide, alkali metal hydride, alkali metal carbonate, alkali metal alkoxide, alkaline earth metal carbonate, or an organic base such as a tertiary amine for example triethylamine, or 1,8-diazabicyclo[5.4.0]undec-7-en (DBU). The process is known in general terms from for example Comprehensive Heterocyclic Chemistry, A.R. Katritzky and C.W. Rees, Pergamon Press, Oxford, New York, 1984, Vol.3; Part 2B; ISBN 0-08-030703-5, S. 482.

According to a further feature of the present invention where one of R¹ or R² in formula (I) is (C₁-C₄)alkyl, (C₁-C₄)haloalkyl, (C₃-C₄)alkenyl, (C₃-C₄)haloalkenyl, (C₃-C₄)alkynyl or (C₃-C₄)haloalkynyl, invention compounds of formula (I) may be prepared by the reaction of the corresponding compound of formula (I) wherein said R¹ or R² respectively is H, and the other radicals and the configurations are as defined in formula (I) (= compounds of the formula (I'), see process claim 7), with an alkylating agent of formula (VI) or (VII) respectively:

R¹-L² (VI)

R²-L² (VII)

wherein L² is a leaving group, generally halogen, preferably chlorine, bromine or iodine, or an alkyl- or phenyl-sulfonyloxy moiety such as methyl sulfonyloxy or 4-toluenesulfonyloxy. Depending on the amounts and reactivity of reactants used and the reactivity of the product, the compound of formula (I) is then the product of a mono- or dialkylation.The reaction is generally performed in an inert solvent such as tetrahydrofuran, dioxan, acetonitrile or N,N-dimethylformamide at a temperature of from 0°C to the reflux temperature of the solvent, preferably at 20°C to 100°C.

According to a further feature of the present invention compounds of formula (I), wherein one of R¹ or R² is an acyl radical, may be prepared by the reaction of the corresponding compound of formula (I) wherein said R¹ or R² respectively is H, and the other radicals and the configurations are as defined in formula (I) or formula (Ia), respectively, with an acylating agent of formula (VIII) or (IX) respectively:

R¹-L³ (VIII)

R²-L³ (IX)

wherein R¹ and R² are each an acyl radical as defined in formula (I) and L³ is a leaving group, generally halogen, preferably chlorine; or with a formylating agent such as formic acid-acetic anhydride. A base is optionally used for the acylation reaction and is generally chosen from an alkali metal hydroxide, alkali metal hydride, alkali metal carbonate, alkali metal alkoxide, alkaline earth metal carbonate, or an organic base such as a tertiary amine for example triethylamine.

Depending on the amounts and reactivity of reactants used, the compound of formula (I) is then the product of a mono- or diacylation.

The reaction is generally performed in an inert solvent such as tetrahydrofuran, dioxan, acetonitrile or N,N-dimethylformamide at a temperature of from 0°C to the reflux temperature of the solvent, preferably at 20°C to 100°C.

The alkylation and acylation reactions can be combined for the preparation of compounds (I) where one of the groups R¹ or R² is (C₁-C₄)alkyl, (C₁-C₄)haloalkyl, (C₃-C₄)alkenyl, and the other one is an acyl group.

Intermediates of formula (III) or (IIIa) may be prepared by the reaction of a compound of formula (X) 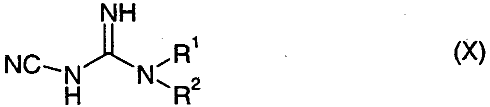 with a compound of said formula (V). The reaction is generally performed using an acid addition salt for example the hydrochloride salt of the compound of formula (X), in a solvent such as 1,2-dichlorobenzene, decalin or white mineral oil, at a temperature of from 20°C to the reflux temperature of the solvent, preferably at 50°C to 200°C.

Intermediates of formula (V) or (Va) may be prepared according to known methods, for example by the reductive amination, optionally asymmetric reductive amination, of ketones of formula (XI) or the corresponding oximes: 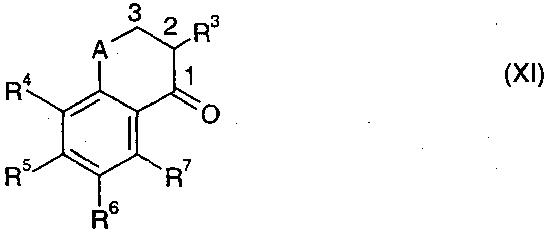 in which R³, R⁴, R⁵, R⁶, R⁷ and A are as defined in formula (I), or the reaction of compounds of formula (XII) 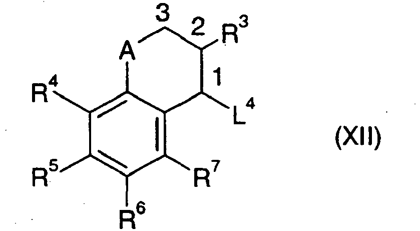 where R³, R⁴, R⁵,R⁶, R⁷ and A are as defined in the compound of formula (I) to be prepared and L⁴ is a leaving group such as halogen, hydroxy, methylsulfonyloxy or 4-toluenesulfonyloxy, with ammonia or a salt thereof, according to known procedures for example as described in patent publication number WO-A-97/031904.

According to a further feature of the present invention said compounds of formula (I), or preferably (Ia), may also be prepared by the reaction of a compound of general formula (IV), in which R¹ and R² are as defined in the compound of formula (I) and L¹ is NH₂ with a compound of formula (XII). Suitable compounds of formula (IV), where L¹ is NH₂, can be prepared analogously to known methods. For example, fluoroalkyl-diamino-s-triazines can be prepared as described by Kretov, A. E.; Davydov, A. V, in Khim. Geterotsikl. Soedin. 1966, 122 or Zh. Obschch. Khim. 1965, 35, 1156 or by other known methods.

Furthermore compounds according formula (I) with R¹ being an amino group can be converted into the compounds bearing a formyl-amino group at this position by heating the educt together with orthoesters in a inert solvent. The reaction can also be performed in a microwave system like the SmithSynthesizier microwave system, available from Biotage, 1725 Discovery Drive, Charlottesville, VA 22911, USA. The process is general known, for example see T. Chancellor, C. Morton; Synthesis (1994) 10, 1023-1025.

For the preparation of compounds of formula (I), in which one or more asymmetric carbon atoms is present as a single enantiomeric form, the above processes can be adapted by employing the appropriate enantiomeric or diastereomeric form of the compounds of formula (III), (IV), (V), (VI), (VII) or (XII).

The preparation of compounds of formula (I) in resolved form or partially resolved form may, for example, be carried out by following the above-defined processes using one or more intermediates (II), (III), (IV) or (V) whose configuration differs from the configuration as defined in the compound of formula (I) to be prepared, and resolving the mixture obtained according to known methods of resolution.

It is generally possible to use customary methods for optical resolutions (cf. Textbooks of Stereochemistry), for example following processes for separating mixtures into diastereomers, for example physical processes, such as crystallization, chromatographic processes, in particular column chromatography and high pressure liquid chromatography, distillation, if appropriate under reduced pressure, extraction and other processes, it is possible to separate the remaining mixtures of enantiomers, generally by chromatographic separation on chiral solid phases. Suitable for preparative amounts or use on an industrial scale are processes such as the crystallization of diastereomeric salts which can be obtained from the compounds (I) using optically active acids and, if appropriate, provided that acidic groups are present, using optically active bases.

Optically active acids which are suitable for optical resolution by crystallization of diastereomeric salts are, for example, camphorsulfonic acid, camphoric acid, bromocamphorsulfonic acid, quinic acid, tartaric acid, dibenzoyltartaric acid and other analogous acids; suitable optically active bases are, for example, quinine, cinchonine, quinidine, brucine, 1-phenylethylamine and other analogous bases.

The crystallizations are then in most cases carried out in aqueous or aqueous-organic solvents, where the diastereomer which is less soluble precipitates first, if appropriate after seeding. One enantiomer of the compound of the formula (I) is then liberated from the precipitated salt, or the other is liberated from the crystals, by acidification or using base.

Enantiomerically pure amine intermediates of formula (V) may be prepared using known methods, for example as described in Houben-Weyl, Methoden der Organischen Chemie, 4. Auflage, Band E 21 b, 1833 ff. or Band E 21 e, 5133. One of the preferred procedures is the reductive amination of ketones of formula (XI) with asymmetric catalysis. Another procedure for the preparation of enantiomerically pure amines of formula (V), is the racemate cleaving method described in J. Prakt. Chem. 339, (1997), pages 381 - 384, or a procedure described in Org. Lett., Vol 3, Nr. 25, page 4101. In this procedure the racemic amine of the general formula (V) is acylated enantioselectively with an optionally substituted fatty acid ester (preferably methyl- or ethyl- chloroacetate or methyl- or ethylmethoxyacetate) in the presence of a biocatalyst. The non acylated enantiomer is then separated by simple treatment with mineral acid. The acylated amine enantiomer is then cleaved back to the corresponding amine, using a base for example an alkali metal hydroxide such as sodium hydroxide, or an acid, for example a mineral acid such as hydrogen chloride.

As biocatalysts, lipases, for example *Pseudomonas cepacia, Candida cylindracea or Candida antarctica,* are particularly suitable for this purpose. Certain of these lipases are also commercially available in immobilized form (brand name: "Novozym 435").

The following acids, for example, are suitable for preparing the acid addition salts of the compounds of the formula (I): hydrohalic acids, such as hydrochloric acid or hydrobromic acid, furthermore phosphoric acid, nitric acid, sulfuric acid, mono- or bifunctional carboxylic acids and hydroxycarboxylic acids, such as acetic acid, oxalic acid, maleic acid, succinic acid, fumaric acid, tartaric acid, citric acid, salicylic acid, sorbic acid or lactic acid, and also sulfonic acids, such as p-toluenesulfonic acid and 1,5-naphthalenedisulfonic acid. The acid addition compounds of the formula (I) can be obtained in a simple manner by the customary methods for forming salts, for example by dissolving a compound of the formula (I) in a suitable organic solvent, such as, for example, methanol, acetone, methylene chloride or benzene, and adding the acid at temperatures from 0 to 100°C, and they can be isolated in a known manner, for example by filtration, and, if appropriate, purified by washing with an inert organic solvent.

The base addition salts of the compounds of the formula (I) are preferably prepared in inert polar solvents such as, for example, water, methanol or acetone at temperature from 0 to 100°C. Examples of suitable bases for preparing the salts according to the invention are alkali metal carbonates, such as potassium carbonate, alkali metal and alkaline earth metal hydroxides, for example NaOH or KOH, alkali metal and alkaline earth metal hydrides, for example NaH, alkali metal and alkaline earth metal alkoxides, for example sodium methoxide, potassium tert-butoxide, or ammonia or ethanolamine. Quaternary ammonium salts can be obtained, for example, by salt exchange or condensation with quaternary ammonium salts of the formula [NRR'R"R"']⁺X⁻ where R, R', R" and R"' independently of one another are (C₁-C₄)alkyl, phenyl or benzyl and X⁻ is an anion, for example Cl⁻ or OH⁻.

Some of the compounds of formula (III) and (V) in which one or more asymmetric carbon atoms are present as a single or optically enriched enantiomeric form are novel and as such form a further feature of the invention, and may be prepared as described above.

Compounds of formula (II), (IV), (VI), (VII), (VIII), (IX), (X), (XI) and (XII), as well as racemic (III) and (V) are known or may be prepared according to known methods.

A collection of compounds of formula (I) which can be synthesized by the abovementioned processes can additionally be prepared in parallel fashion, which can be effected manually, partly automated or fully automated. In this context, it is possible to automate the procedure of the reaction, work-up or purification of the products or intermediates. In total, this is to be understood as meaning a procedure which is described, for example, by S. H. DeWitt in "Annual Reports in Combinatorial Chemistry and Molecular Diversity: Automated Synthesis", Volume 1, published by Escom, 1997, pages 69 to 77.

For carrying out the reaction and work-up in parallel fashion, a series of commercially available apparatuses can be used as they are available from, for example, Stem Corporation, Woodrolfe Road, Tollesbury, Essex, CM9 8SE, England or H + P Labortechnik AG, a unit of Thermo Electronic, Bruckmannring 15-19, 85764 Oberschleissheim, Germany. To carry out the parallel purification of compounds (I) or of intermediates obtained during the preparation, there are available, inter alia, chromatographic equipment, for example from Teledyne ISCO, Inc., 4700 Superior Street, Lincoln, NE 68504, USA. The equipment mentioned makes possible a modular procedure, where the individual steps are automated, but manual operation has to be carried out between the steps. This can be circumvented by employing partly or fully integrated automation systems, in which the automation modules in question are operated by, for example, robots. Such automation systems can be obtained from, for example, Caliper Life Sciences Worldwide Headquarters 68 Elan Street, Hopkinton, MA 01748, USA (former Zymark Corporation).

In addition to the above-described methods, compounds of formula (I) can be prepared in full or partly by solid-phase supported methods. To this end, individual intermediates or all intermediates of the synthesis or of a synthesis adapted to the procedure in question are bound to a synthesis resin. Solid-phase supported synthetic methods are described extensively in the specialist literature, for example: Barry A. Bunin in "The Combinatorial Index",_ published by Academic Press, 1998. The use of solid-phase supported synthesis methods permits a series of protocols known from the literature which, in turn, can be carried out manually or in an automated fashion. For example, the "teabag method" (Houghten, US 4,631,211; Houghten et al., Proc. Natl. Acad. Sci., 1985, 82, 5131 - 5135) can be partly automated with products of IRORI CombiChem-line available from NEXUS Biosystems, 12140, Community Road, Poway, CA 92064, or also from IRORI, 11149 North Torrey Pines Road, La Jolla, CA 92037, USA. Solid-phase supported parallel synthesis can be automated successfully for example using equipment of the company Argonaut Technologies, Inc., 887 Industrial Road, San Carlos, CA 94070, USA, or of the company Biotage, 1725 Discovery Drive, Charlottesville, VA 22911, USA or of the company MultiSynTech GmbH, Wullener Feld 4, 58454 Witten, Germany.

The preparation in accordance with the processes described herein yields compounds of formula (I) in the form of substance collections or substance libraries. Subject matter of the present invention are therefore also libraries of the compounds of formula (I) which contain at least two compounds of formula (I), and of their precursors.

The following non-limiting Examples illustrate the preparation of the compounds of formula (I).

### A. Chemical Examples

In the Examples which follow, quantities (also percentages) are weight based unless stated otherwise. Ratios of solvents are volume based unless stated otherwise.

### Example A1

### 6-(Difluoromethyl)-N-[(4R)-8-fluoro-3,4-dihydro-2H-chromen-4-yl]-1,3,5-triazine-2,4-diamine (Table 2, Compound number 2.206)

A 25 % solution of sodium methoxide (0.94 g, 0.99 ml, 0.0043 mol) in methanol was added to a stirred suspension of amino-N-[{[(4R)-8-fluoro-3,4-dihydro-2H-chromen-4-yl]-amino}(imino)methyl]iminomethanaminium chloride (a biguanide of formula (IIIa)) (0.5 g, 0.0017 mol) in methanol. Then ethyl difluroacetate (0.65 g, 0.52 ml, 0.0052 mol) was added. After 4 hours the mixture was filtered, and the filtrate evaporated. The residue was dissolved in ethyl acetate, washed (water), dried (sodium sulfate) and evaporated. The residue was purified by column chromatography, eluting with a mixture of ethyl acetate:heptane (7:3) as eluent to give 6-(difluoromethyl)-N-[(4R)-8-fluoro-3,4-dihydro-2H-chromen-4-yl]-1,3,5-triazine-2,4-diamine (solid foam, 0.298 g, purity app. 90%, yield 50%).

### Example A2

### 6-(difluoromethyl)-N-[(1 R,2S)-2,6-dimethyl-2,3-dihydro-1 H-inden-1-yl]-1,3,5-triazine-2,4-diamine (Table 3, Compound number 3.1)

A 25 % solution of sodium methoxide (1.08 g, 1.14 ml, 0.0050 mol) in methanol was added to a stirred suspension of (1*R*,2*S*)-1-(bisguanidino)-2,6-dimethylindane monohydrochloride (0.564 g, 0.0020 mol) in methanol. Then ethyl difluoroacetate (0.75 g, 0.0060 mol) was added. After 4 hours the mixture was filtered, and the filtrate evaporated. The residue was dissolved in ethyl acetate, washed (water), dried (sodium sulfate) and evaporated. The residue was purified by column chromatography, eluting with a mixture of ethyl acetate:heptane (7:3) as eluent to give 6-(difluoromethyl)-N-[(1R,2S)-2,6-dimethyl-2,3-dihydro-1H-inden-1-yl]-1,3,5-triazine-2,4-diamine (0.491 g, yield 76%), mp: 132-134 °C, optical rotation (chloroform, c=1): +161.0 °, purity 95 % (hplc, Chiralcel OD, 250X4.6 mm, eluent n-hexane:2-propanol 90:10, rf 17.93 min); the optical purity obtained was at least 90 % ee. (ee % = enantiomeric excess in %).

### Example A3

### N-(4-(difluoromethyl)-6-{[(1R,2S)-2,6-dimethyl-2,3-dihydro-1H-inden-1-yl]-amino}-1,3,5-triazin-2-yl)propanamide (Table 3, Compound number 3.109)

A stirred suspension of 6-(difluoromethyl)-N-[(1 R,2S)-2,6-dimethyl-2,3-dihydro-1H-inden-1-yl]-1,3,5-triazine-2,4-diamine (0.300 g, 0.0009 mol) (see example A2) in propanoic anhydride (3 ml) is heated for 60 minutes to 120°C. After cooling the reaction mixture was purified by column chromatography, eluting with a mixture of ethyl acetate:heptane (7:3) as eluent to give N-(4-(difluoromethyl)-6-{[(1 R,2S)-2,6-dimethyl-2,3-dihydro-1H-inden-1-yl]amino}-1,3,5-triazin-2-yl)propanamide (0.145 g, yield 43%), mp. 66°C.

The following preferred compounds of formula (I) or (Ia) shown in Tables 1 to 4 also form part of the present invention, and are obtained by, or analogously to, the above Examples A1, A2 and A3 or the above-described general methods.

### Example A4

### N-[-4-(difluoromethyl)-6-{[(1R,WS)-2,6-dimethyl-2,3-dihydro-1H-inden-1-yl]amino}-1,3,5-triazin-2-yl]formamide

A mixture of 6-(difluoromethyl)-N-[(1 R,2S)-2,6-dimethyl-2,3-dihydro-1 H-inden-1-yl]-1,3,5-triazine-2,4-diamine (0.25 g, 0.0008 Mol) and triethylorthoformiate (1.46 g, 1.63 ml, 0.0098 Mol) in acetanhydride (0.5 g, 0,46 ml, 0.0049 Mol) has been heated for 30 minutes to 160°C (capped vial, SmithSynthesizer microwave system, available at Biotage, 1725 Discovery Drive, Charlottesville, VA 22911, USA). After cooling, the reaction mixture was purified by column chromatography, eluting with a mixture of ethyl acetate:heptane (7:3) as eluent to give N-[4-(difluoromethyl)-6-{[(1R,2S)-2,6-dimethyl-2,3-dihydro-1H-inden-1-yl]amino}-1,3,5-triazin-2-yl]-formamide (waxy, 0.159 g, purity app. 90 %, yield 50 %), mp. 66°C.

The following abbreviations are used in the Tables 1 to 4:
- Me: = methyl
- Et: = ethyl
- n-Prop: = n-propyl
- i-Prop: = isopropyl
- n-Bu: = n-butyl
- sec-Bu: = sec-butyl
- i-Bu: = isobutyl
- t-Bu: = tert-butyl
- -: = direct bond in the definition for A
- Cpd: = Compound Number. Compound numbers are given for reference purposes only
- Ref.: = reference to data explained at the end of the table
- optical purity: = The optically active compounds were obtained in an optical purity of at least 90 % ee (ee % = enantiomeric excess in %)

**Table 1: Compounds of formula (I) (mixture of stereoisomers, racemic)**

| | | | | | | | | | |
|---|---|---|---|---|---|---|---|---|---|
| 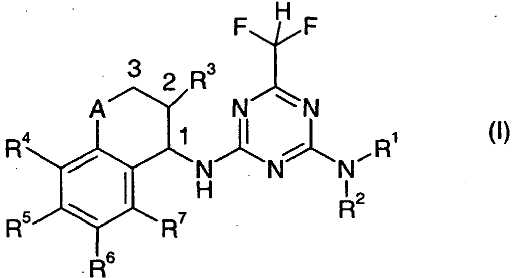 | | | | | | | | | |

| **Cpd** | **R¹** | **R²** | **R³** | **R⁴** | **R⁵** | **R⁶** | **R⁷** | **A** | **Ref.** |
|---|---|---|---|---|---|---|---|---|---|
| 1.1 | H | H | H | H | H | Me | H | - | |
| 1.2 | H | H | H | H | H | Me | H | CH₂ | |
| 1.3 | H | H | H | H | H | Me | H | O | |
| 1.4 | H | H | H | Me | H | Me | H | - | |
| 1.5 | H | H | H | Me | H | Me | H | CH₂ | Ref. |
| 1.6 | H | H | H | Me | H | Me | H | O | |
| 1.7 | H | H | Me | H | H | Me | H | - | |
| 1.8 | H | H | Me | H | H | Me | H | CH₂ | |
| 1.9 | H | H | Me | H | H | Me | H | O | |
| 1.10 | H | H | Me | Me | H | Me | H | - | |
| 1.11 | H | H | Me | Me | H | Me | H | CH₂ | |
| 1.12 | H | H | Me | Me | H | Me | H | O | |
| 1.13 | H | H | H | H | Me | Me | H | - | |
| 1.14 | H | H | H | H | Me | Me | H | CH₂ | |
| 1.15 | H | H | H | H | Me | Me | H | O | |
| 1.16 | H | H | H | Me | Me | Me | H | - | |
| 1.17 | H | H | H | Me | Me | Me | H | CH₂ | |
| 1.18 | H | H | H | Me | Me | Me | H | O | |
| 1.19 | H | H | Me | H | Me | Me | H | - | |
| 1.20 | H | H | Me | H | Me | Me | H | CH₂ | |
| 1.21 | H | H | Me | H | Me | Me | H | O | |
| 1.22 | H | H | Me | Me | Me | Me | H | - | |
| 1.23 | H | H | Me | Me | Me | Me | H | CH₂ | |
| 1.24 | H | H | Me | Me | Me | Me | H | O | |
| 1.25 | H | H | H | H | H | Et | H | - | |
| 1.26 | H | H | H | H | H | Et | H | CH₂ | |
| 1.27 | H | H | H | H | H | Et | H | O | |
| 1.28 | H | H | H | Me | H | Et | H | - | |
| 1.29 | H | H | H | Me | H | Et | H | CH₂ | |
| 1.30 | H | H | H | Me | H | Et | H | O | |
| 1.31 | H | H | Me | H | H | Et | H | - | |
| 1.32 | H | H | Me | H | H | Et | H | CH₂ | |
| 1.33 | H | H | Me | H | H | Et | H | O | |
| 1.34 | H | H | Me | Me | H | Et | H | - | |
| 1.35 | H | H | Me | Me | H | Et | H | CH₂ | |
| 1.36 | H | H | Me | Me | H | Et | H | O | |
| 1.37 | H | H | H | H | Me | Et | H | - | |
| 1.38 | H | H | H | H | Me | Et | H | CH₂ | |
| 1.39 | H | H | H | H | Me | Et | H | O | |
| 1.40 | H | H | H | Me | Me | Et | H | - | |
| 1.41 | H | H | H | Me | Me | Et | H | CH₂ | |
| 1.42 | H | H | H | Me | Me | Et | H | O | |
| 1.43 | H | H | Me | H | Me | Et | H | - | |
| 1.44 | H | H | Me | H | Me | Et | H | CH₂ | |
| 1.45 | H | H | Me | H | Me | Et | H | O | |
| 1.46 | H | H | Me | Me | Me | Et | H | - | |
| 1.47 | H | H | Me | Me | Me | Et | H | CH₂ | |
| 1.48 | H | H | Me | Me | Me | Et | H | O | |
| 1.49 | H | H | OMe | H | H | Me | H | - | |
| 1.50 | H | H | OMe | H | H | Me | H | CH₂ | |
| 1.51 | H | H | OMe | H | H | Me | H | O | |
| 1.52 | H | H | OMe | Me | H | Me | H | - | |
| 1.53 | H | H | OMe | Me | H | Me | H | CH₂ | |
| 1.54 | H | H | OMe | Me | H | Me | H | O | |
| 1.55 | H | H | Et | H | H | Me | H | - | |
| 1.56 | H | H | Et | H | H | Me | H | CH₂ | |
| 1.57 | H | H | Et | H | H | Me | H | O | |
| 1.58 | H | H | Et | Me | H | Me | H | - | |
| 1.59 | H | H | Et | Me | H | Me | H | CH₂ | |
| 1.60 | H | H | Et | Me | H | Me | H | O | |
| 1.61 | H | H | OMe | H | Me | Me | H | - | |
| 1.62 | H | H | OMe | H | Me | Me | H | CH₂ | |
| 1.63 | H | H | OMe | H | Me | Me | H | O | |
| 1.64 | H | H | OMe | Me | Me | Me | H | - | |
| 1.65 | H | H | OMe | Me | Me | Me | H | CH₂ | |
| 1.66 | H | H | OMe | Me | Me | Me | H | O | |
| 1.67 | H | H | Et | H | Me | Me | H | - | |
| 1.68 | H | H | Et | H | Me | Me | H | CH₂ | |
| 1.69 | H | H | Et | H | Me | Me | H | O | |
| 1.70 | H | H | Et | Me | Me | Me | H | - | |
| 1.71 | H | H | Et | Me | Me | Me | H | CH₂ | |
| 1.72 | H | H | Et | Me | Me | Me | H | O | |
| 1.73 | H | H | H | Me | Me | F | H | - | |
| 1.74 | H | H | H | Me | Me | Me | H | CH₂ | |
| 1.75 | H | H | H | Me | F | Me | H | O | |
| 1.76 | H | H | H | Me | H | Me | H | - | |
| 1.77 | H | H | H | Me | H | Me | H | CH₂ | |
| 1.78 | H | H | H | Me | H | Br | H | O | |
| 1.79 | H | H | H | Me | H | Me | Me | | |
| 1.80 | H | H | H | Me | H | Me | Me | CH₂ | |
| 1.81 | H | H | H | Me | H | Me | Me | O | |
| 1.82 | H | H | H | Me | Me | Me | Me | | |
| 1.83 | H | H | H | Me | Me | Me | Me | CH₂ | |
| 1.84 | H | H | H | Me | Me | Me | Me | O | |
| 1.85 | H | H | H | H | H | n-Prop | H | - | |
| 1.86 | H | H | H | H | H | n-Prop | H | CH₂ | |
| 1.87 | H | H | H | H | H | n-Prop | H | O | |
| 1.88 | H | H | H | H | H | i-Prop | H | - | |
| 1.89 | H | H | H | H | H | i-Prop | H | CH₂ | |
| 1.90 | H | H | H | H | H | i-Prop | H | O | |
| 1.91 | H | H | Me | H | H | i-Prop | H | - | |
| 1.92 | H | H | Me | H | H | i-Prop | H | CH₂ | |
| 1.93 | H | H | Me | H | H | i-Prop | H | O | |
| 1.94 | H | H | Et | H | H | i-Prop | H | - | |
| 1.95 | H | H | Et | H | H | i-Prop | H | CH₂ | |
| 1.96 | H | H | Et | H | H | i-Prop | H | O | |
| 1.97 | H | H | H | H | H | n-Bu | H | - | |
| 1.98 | H | H | H | H | H | n-Bu | H | CH₂ | |
| 1.99 | H | H | H | H | H | n-Bu | H | O | |
| 1.100 | H | H | H | H | H | sec-Bu | H | - | |
| 1.101 | H | H | H | H | H | sec-Bu | H | CH₂ | |
| 1.102 | H | H | H | H | H | sec-Bu | H | O | |
| 1.103 | H | H | H | H | H | OMe | H | - | |
| 1.104 | H | H | H | H | H | OMe | H | CH₂ | |
| 1.105 | H | H | H | H | H | OMe | H | O | |
| 1.106 | H | H | Me | H | H | OMe | H | - | |
| 1.107 | H | H | Me | H | H | OMe | H | CH₂ | |
| 1.108 | H | H | Me | H | H | OMe | H | O | |
| 1.109 | H | H | Et | H | H | OMe | H | - | |
| 1.110 | H | H | Et | H | H | OMe | H | CH₂ | |
| 1.111 | H | H | Et | H | H | OMe | H | O | |
| 1.112 | H | H | H | H | H | OEt | H | - | |
| 1.113 | H | H | H | H | H | OEt | H | CH₂ | |
| 1.114 | H | H | H | H | H | OEt | H | O | |
| 1.115 | H | H | Me | H | H | OEt | H | - | |
| 1.116 | H | H | Me | H | H | OEt | H | CH₂ | |
| 1.117 | H | H | Me | H | H | OEt | H | O | |
| 1.118 | H | H | Et | H | H | OEt | H | - | |
| 1.119 | H | H | Et | H | H | OEt | H | CH₂ | |
| 1.120 | H | H | Et | H | H | OEt | H | O | |
| 1.121 | H | H | H | H | H | CN | H | - | |
| 1.122 | H | H | H | H | H | CN | H | CH₂ | |
| 1.123 | H | H | H | H | H | CN | H | O | |
| 1.124 | H | H | Me | H | H | CN | H | - | |
| 1.125 | H | H | Me | H | H | CN | H | CH₂ | |
| 1.126 | H | H | Me | H | H | CN | H | O | |
| 1.127 | H | H | H | H | H | C≡CMe | H | - | |
| 1.128 | H | H | H | H | H | C≡CMe | H | CH₂ | |
| 1.129 | H | H | H | H | H | C≡CMe | H | O | |
| 1.130 | H | H | Me | H | H | C≡CMe | H | - | |
| 1.131 | H | H | Me | H | H | C≡CMe | H | CH₂ | |
| 1.132 | H | H | Me | H | H | C≡CMe | H | O | |
| 1.133 | H | H | H | H | H | C≡CH | H | - | |
| 1.134 | H | H | H | H | H | C≡CH | H | CH₂ | |
| 1.135 | H | H | H | H | H | C≡CH | H | O | |
| 1.136 | H | H | Me | H | H | C≡CH | H | - | |
| 1.137 | H | H | Me | H | H | C≡CH | H | CH₂ | |
| 1.138 | H | H | Me | H | H | C≡CH | H | O | |
| 1.139 | H | H | H | H | H | F | H | - | |
| 1.140 | H | H | H | H | H | F | H | CH₂ | |
| 1.141 | H | H | H | H | H | F | H | O | |
| 1.142 | H | H | Me | H | H | F | H | - | |
| 1.143 | H | H | Me | H | H | F | H | CH₂ | |
| 1.144 | H | H | Me | H | H | F | H | O | |
| 1.145 | H | H | H | H | H | Cl | H | - | |
| 1.146 | H | H | H | H | H | Cl | H | CH₂ | |
| 1.147 | H | H | H | H | H | Cl | H | O | |
| 1.148 | H | H | Me | H | H | Cl | H | - | |
| 1.149 | H | H | Me | H | H | Cl | H | CH₂ | |
| 1.150 | H | H | Me | H | H | Cl | H | O | |
| 1.151 | H | H | H | H | H | Br | H | - | |
| 1.152 | H | H | H | H | H | Br | H | CH₂ | |
| 1.153 | H | H | H | H | H | Br | H | O | |
| 1.154 | H | H | Me | H | H | Br | H | - | |
| 1.155 | H | H | Me | H | H | Br | H | CH₂ | |
| 1.156 | H | H | Me | H | H | Br | H | O | |
| 1.157 | H | H | H | H | H | I | H | - | |
| 1.158 | H | H | H | H | H | I | H | CH₂ | |
| 1.159 | H | H | H | H | H | I | H | O | |
| 1.160 | H | H | Me | H | H | I | H | - | |
| 1.161 | H | H | Me | H | H | I | H | CH₂ | |
| 1.162 | H | H | Me | H | H | I | H | O | |
| 1.163 | H | H | OCH₂C≡CH | H | H | Me | H | - | |
| 1.164 | H | H | OCH₂C≡CH | H | H | Me | H | CH₂ | |
| 1.165 | H | H | OCH₂C≡CH | H | H | Me | H | O | |
| 1.166 | H | H | Me | H | H | OCH₂C≡CH | H | - | |
| 1.167 | H | H | Me | H | H | OCH₂C≡CH | H | CH₂ | |
| 1.168 | H | H | Me | H | H | OCH₂C≡CH | H | O | |
| 1.169 | H | H | C≡CMe | H | H | Me | H | - | |
| 1.170 | H | H | C≡CMe | H | H | Me | H | CH₂ | |
| 1.171 | H | H | C≡CMe | H | H | Me | H | O | |
| 1.172 | Me | H | Me | H | H | Me | H | - | |
| 1.173 | Me | H | Me | H | H | Me | H | CH₂ | |
| 1.174 | Me | H | Me | H | H | Me | H | O | |
| 1.175 | Me | H | H | H | H | Me | H | - | |
| 1.176 | Me | H | H | H | H | Me | H | CH₂ | |
| 1.177 | Me | H | H | H | H | Me | H | O | |
| 1.178 | Me | Me | Me | H | H | Me | H | - | |
| 1.179 | Me | Me | Me | H | H | Me | H | CH₂ | |
| 1.180 | Me | Me | Me | H | H | Me | H | O | |
| 1.181 | Et | H | Me | H | H | Me | H | - | |
| 1.182 | Et | H | Me | H | H | Me | H | CH₂ | |
| 1.183 | Et | H | Me | H | H | Me | H | O | |
| 1.184 | C(O)Me | H | H | H | H | Me | H | - | |
| 1.185 | C(O)Me | H | H | H | H | Me | H | CH₂ | |
| 1.186 | C(O)Me | H | H | H | H | Me | H | O | |
| 1.187 | C(O)Me | H | Me | H | H | Me | H | - | |
| 1.188 | C(O)Me | H | Me | H | H | Me | H | CH₂ | |
| 1.189 | C(O)Me | H | Me | H | H | Me | H | O | |
| 1.190 | C(O)Et | H | H | H | H | Me | H | - | |
| 1.191 | C(O)Et | H | H | H | H | Me | H | CH₂ | |
| 1.192 | C(O)Et | H | H | H | H | Me | H | O | |
| 1.193 | C(O)Et | H | Me | H | H | Me | H | - | |
| 1.194 | C(O)Et | H | Me | H | H | Me | H | CH₂ | |
| 1.195 | C(O)Et | H | Me | H | H | Me | H | O | |
| 1.196 | C(O)-t-Bu | H | Me | H | H | Me | H | - | |
| 1.197 | C(O)-t-Bu | H | Me | H | H | Me | H | CH₂ | |
| 1.198 | C(O)-t-Bu | H | Me | H | H | Me | H | O | |
| 1.199 | C(O)CF₃ | H | H | H | H | Me | H | - | |
| 1.200 | C(O)CF₃ | H | H | H | H | Me | H | CH₂ | |
| 1.201 | C(O)CF₃ | H | H | H | H | Me | H | O | |
| 1.202 | C(O)CF₃ | H | Me | H | H | Me | H | - | |
| 1.203 | C(O)CF₃ | H | Me | H | H | Me | H | CH₂ | |
| 1.204 | C(O)CF₃ | H | Me | H | H | Me | H | O | |
| 1.205 | H | H | H | Me | H | H | H | O | Ref. |
| 1.206 | H | H | H | F | H | H | H | O | |
| 1.207 | H | H | H | H | H | t-Bu | H | - | Ref. |

| | | | | | | | | | |
|---|---|---|---|---|---|---|---|---|---|
| References to compounds (Ref.) in Table 1: Compound number 1.5: obtained as a waxy material Compound number 1.205: obtained as a waxy material Compound number 1.207: solid, melting point 214.6°C | | | | | | | | | |

**Table 2: Compounds of formula (Ia) (optically actice compounds)**

| | | | | | | | | | |
|---|---|---|---|---|---|---|---|---|---|
| 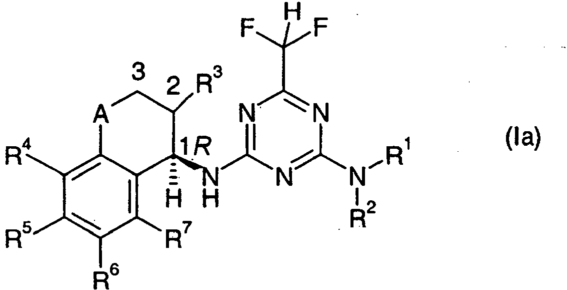 | | | | | | | | | |

| **Cpd** | **R¹** | **R²** | **R³** | **R⁴** | **R⁵** | **R⁶** | **R⁷** | **A** | **Ref.** |
|---|---|---|---|---|---|---|---|---|---|
| 2.1 | H | H | H | H | H | Me | H | - | Ref. |
| 2.2 | H | H | H | H | H | Me | H | CH₂ | Ref. |
| 2.3 | H | H | H | H | H | Me | H | O | Ref. |
| 2.4 | H | H | H | Me | H | Me | H | - | |
| 2.5 | H | H | H | Me | H | Me | H | CH₂ | Ref. |
| 2.6 | H | H | H | Me | H | Me | H | O | |
| 2.7 | H | H | Me | H | H | Me | H | - | |
| 2.8 | H | H | Me | H | H | Me | H | CH₂ | |
| 2.9 | H | H | Me | H | H | Me | H | O | |
| 2.10 | H | H | Me | Me | H | Me | H | - | |
| 2.11 | H | H | Me | Me | H | Me | H | CH₂ | |
| 2.12 | H | H | Me | Me | H | Me | H | O | |
| 2.13 | H | H | H | H | Me | Me | H | - | |
| 2.14 | H | H | H | H | Me | Me | H | CH₂ | |
| 2.15 | H | H | H | H | Me | Me | H | O | |
| 2.16 | H | H | H | Me | Me | Me | H | - | |
| 2.17 | H | H | H | Me | Me | Me | H | CH2 | |
| 2.18 | H | H | H | Me | Me | Me | H | O | |
| 2.19 | H | H | Me | H | Me | Me | H | - | |
| 2.20 | H | H | Me | H | Me | Me | H | CH₂ | |
| 2.21 | H | H | Me | H | Me | Me | H | O | |
| 2.22 | H | H | Me | Me | Me | Me | H | - | |
| 2.23 | H | H | Me | Me | Me | Me | H | CH₂ | |
| 2.24 | H | H | Me | Me | Me | Me | H | O | |
| 2.25 | H | H | H | H | H | Et | H | - | Ref. |
| 2.26 | H | H | H | H | H | Et | H | CH₂ | |
| 2.27 | H | H | H | H | H | Et | H | O | |
| 2.28 | H | H | H | Me | H | Et | H | - | |
| 2.29 | H | H | H | Me | H | Et | H | CH₂ | |
| 2.30 | H | H | H | Me | H | Et | H | O | |
| 2.31 | H | H | Me | H | H | Et | H | - | |
| 2.32 | H | H | Me | H | H | Et | H | CH₂ | |
| 2.33 | H | H | Me | H | H | Et | H | O | |
| 2.34 | H | H | Me | Me | H | Et | H | - | |
| 2.35 | H | H | Me | Me | H | Et | H | CH₂ | |
| 2.36 | H | H | Me | Me | H | Et | H | O | |
| 2.37 | H | H | H | H | Me | Et | H | - | |
| 2.38 | H | H | H | H | Me | Et | H | CH₂ | |
| 2.39 | H | H | H | H | Me | Et | H | O | |
| 2.40 | H | H | H | Me | Me | Et | H | - | |
| 2.41 | H | H | H | Me | Me | Et | H | CH₂ | |
| 2.42 | H | H | H | Me | Me | Et | H | O | |
| 2.43 | H | H | Me | H | Me | Et | H | - | |
| 2.44 | H | H | Me | H | Me | Et | H | CH₂ | |
| 2.45 | H | H | Me | H | Me | Et | H | O | |
| 2.46 | H | H | Me | Me | Me | Et | H | - | |
| 2.47 | H | H | Me | Me | Me | Et | H | CH₂ | |
| 2.48 | H | H | Me | Me | Me | Et | H | O | |
| 2.49 | H | H | OMe | H | H | Me | H | - | |
| 2.50 | H | H | OMe | H | H | Me | H | CH₂ | |
| 2.51 | H | H | OMe | H | H | Me | H | O | |
| 2.52 | H | H | OMe | Me | H | Me | H | - | |
| 2.53 | H | H | OMe | Me | H | Me | H | CH₂ | |
| 2.54 | H | H | OMe | Me | H | Me | H | O | |
| 2.55 | H | H | Et | H | H | Me | H | - | |
| 2.56 | H | H | Et | H | H | Me | H | CH₂ | |
| 2.57 | H | H | Et | H | H | Me | H | O | |
| 2.58 | H | H | Et | Me | H | Me | H | - | |
| 2.59 | H | H | Et | Me | H | Me | H | CH₂ | |
| 2.60 | H | H | Et | Me | H | Me | H | O | |
| 2.61 | H | H | OMe | H | Me | Me | H | - | |
| 2.62 | H | H | OMe | H | Me | Me | H | CH₂ | |
| 2.63 | H | H | OMe | H | Me | Me | H | O | |
| 2.64 | H | H | OMe | Me | Me | Me | H | - | |
| 2.65 | H | H | OMe | Me | Me | Me | H | CH₂ | |
| 2.66 | H | H | OMe | Me | Me | Me | H | O | |
| 2.67 | H | H | Et | H | Me | Me | H | - | |
| 2.68 | H | H | Et | H | Me | Me | H | CH₂ | |
| 2.69 | H | H | Et | H | Me | Me | H | O | |
| 2.70 | H | H | Et | Me | Me | Me | H | - | |
| 2.71 | H | H | Et | Me | Me | Me | H | CH₂ | |
| 2.72 | H | H | Et | Me | Me | Me | H | O | |
| 2.73 | H | H | H | Me | Me | F | H | - | |
| 2.74 | H | H | H | Me | Me | Me | H | CH₂ | |
| 2.75 | H | H | H | Me | F | Me | H | O | |
| 2.76 | H | H | H | Me | H | Me | H | - | |
| 2.77 | H | H | H | Me | H | Me | H | CH₂ | |
| 2.78 | H | H | H | Me | H | Br | H | O | |
| 2.79 | H | H | H | Me | H | Me | Me | - | |
| 2.80 | H | H | H | Me | H | Me | Me | CH₂ | |
| 2.81 | H | H | H | Me | H | Me | Me | O | |
| 2.82 | H | H | H | Me | Me | Me | Me | - | |
| 2.83 | H | H | H | Me | Me | Me | Me | CH₂ | |
| 2.84 | H | H | H | Me | Me | Me | Me | O | |
| 2.85 | H | H | H | H | H | n-Prop | H | - | |
| 2.86 | H | H | H | H | H | n-Prop | H | CH₂ | |
| 2.87 | H | H | H | H | H | n-Prop | H | O | |
| 2.88 | H | H | H | H | H | i-Prop | H | - | |
| 2.89 | H | H | H | H | H | i-Prop | H | CH₂ | |
| 2.90 | H | H | H | H | H | i-Prop | H | O | |
| 2.91 | H | H | Me | H | H | i-Prop | H | - | |
| 2.92 | H | H | Me | H | H | i-Prop | H | CH₂ | |
| 2.93 | H | H | Me | H | H | i-Prop | H | O | |
| 2.94 | H | H | Et | H | H | i-Prop | H | - | |
| 2.95 | H | H | Et | H | H | i-Prop | H | CH₂ | |
| 2.96 | H | H | Et | H | H | i-Prop | H | O | |
| 2.97 | H | H | H | H | H | n-Bu | H | - | |
| 2.98 | H | H | H | H | H | n-Bu | H | CH₂ | |
| 2.99 | H | H | H | H | H | n-Bu | H | O | |
| 2.100 | H | H | H | H | H | sec-Bu | H | - | |
| 2.101 | H | H | H | H | H | sec-Bu | H | CH₂ | |
| 2.102 | H | H | H | H | H | sec-Bu | H | O | |
| 2.103 | H | H | H | H | H | OMe | H | - | |
| 2.104 | H | H | H | H | H | OMe | H | CH₂ | |
| 2.105 | H | H | H | H | H | OMe | H | O | |
| 2.106 | H | H | Me | H | H | OMe | H | - | |
| 2.107 | H | H | Me | H | H | OMe | H | CH₂ | |
| 2.108 | H | H | Me | H | H | OMe | H | O | |
| 2.109 | H | H | Et | H | H | OMe | H | - | |
| 2.110 | H | H | Et | H | H | OMe | H | CH₂ | |
| 2.111 | H | H | Et | H | H | OMe | H | O | |
| 2.112 | H | H | H | H | H | OEt | H | - | |
| 2.113 | H | H | H | H | H | OEt | H | CH₂ | |
| 2.114 | H | H | H | H | H | OEt | H | O | |
| 2.115 | H | H | Me | H | H | OEt | H | - | |
| 2.116 | H | H | Me | H | H | OEt | H | CH₂ | |
| 2.117 | H | H | Me | H | H | OEt | H | O | |
| 2.118 | H | H | Et | H | H | OEt | H | - | |
| 2.119 | H | H | Et | H | H | OEt | H | CH₂ | |
| 2.120 | H | H | Et | H | H | OEt | H | O | |
| 2.121 | H | H | H | H | H | CN | H | - | |
| 2.122 | H | H | H | H | H | CN | H | CH₂ | |
| 2.123 | H | H | H | H | H | CN | H | O | |
| 2.124 | H | H | Me | H | H | CN | H | - | |
| 2.125 | H | H | Me | H | H | CN | H | CH₂ | |
| 2.126 | H | H | Me | H | H | CN | H | O | |
| 2.127 | H | H | H | H | H | C≡CMe | H | - | |
| 2.128 | H | H | H | H | H | C≡CMe | H | CH₂ | |
| 2.129 | H | H | H | H | H | C≡CMe | H | O | |
| 2.130 | H | H | Me | H | H | C≡CMe | H | - | |
| 2.131 | H | H | Me | H | H | C≡CMe | H | CH₂ | |
| 2.132 | H | H | Me | H | H | C≡CMe | H | O | |
| 2.133 | H | H | H | H | H | C≡CH | H | - | |
| 2.134 | H | H | H | H | H | C≡CH | H | CH₂ | |
| 2.135 | H | H | H | H | H | C≡CH | H | O | |
| 2.136 | H | H | Me | H | H | C≡CH | H | - | |
| 2.137 | H | H | Me | H | H | C≡CH | H | CH₂ | |
| 2.138 | H | H | Me | H | H | C≡CH | H | O | |
| 2.139 | H | H | H | H | H | F | H | - | Ref. |
| 2.140 | H | H | H | H | H | F | H | CH₂ | |
| 2.141 | H | H | H | H | H | F | H | O | |
| 2.142 | H | H | Me | H | H | F | H | - | |
| 2.143 | H | H | Me | H | H | F | H | CH₂ | |
| 2.144 | H | H | Me | H | H | F | H | O | |
| 2.145 | H | H | H | H | H | Cl | H | - | |
| 2.146 | H | H | H | H | H | Cl | H | CH₂ | |
| 2.147 | H | H | H | H | H | Cl | H | O | |
| 2.148 | H | H | Me | H | H | Cl | H | - | |
| 2.149 | H | H | Me | H | H | Cl | H | CH₂ | |
| 2.150 | H | H | Me | H | H | Cl | H | O | |
| 2.151 | H | H | H | H | H | Br | H | - | |
| 2.152 | H | H | H | H | H | Br | H | CH₂ | |
| 2.153 | H | H | H | H | H | Br | H | O | |
| 2.154 | H | H | Me | H | H | Br | H | - | |
| 2.155 | H | H | Me | H | H | Br | H | CH₂ | |
| 2.156 | H | H | Me | H | H | Br | H | O | |
| 2.157 | H | H | H | H | H | I | H | - | |
| 2.158 | H | H | H | H | H | I | H | CH₂ | |
| 2.159 | H | H | H | H | H | I | H | O | |
| 2.160 | H | H | Me | H | H | I | H | - | |
| 2.161 | H | H | Me | H | H | I | H | CH₂ | |
| 2.162 | H | H | Me | H | H | I | H | O | |
| 2.163 | H | H | OCH₂C≡CH | H | H | Me | H | - | |
| 2.164 | H | H | OCH₂C≡CH | H | H | Me | H | CH₂ | |
| 2.165 | H | H | OCH₂C≡CH | H | H | Me | H | O | |
| 2.166 | H | H | Me | H | H | OCH₂C≡CH | H | - | |
| 2.167 | H | H | Me | H | H | OCH₂C≡CH | H | CH₂ | |
| 2.168 | H | H | Me | H | H | OCH₂C≡CH | H | O | |
| 2.169 | H | H | C≡CMe | H | H | Me | H | - | |
| 2.170 | H | H | C≡CMe | H | H | Me | H | CH₂ | |
| 2.171 | H | H | C≡CMe | H | H | Me | H | O | |
| 2.172 | Me | H | Me | H | H | Me | H | - | |
| 2.173 | Me | H | Me | H | H | Me | H | CH₂ | |
| 2.174 | Me | H | Me | H | H | Me | H | O | |
| 2.175 | Me | H | H | H | H | Me | H | - | |
| 2.176 | Me | H | H | H | H | Me | H | CH₂ | |
| 2.177 | Me | H | H | H | H | Me | H | O | |
| 2.178 | Me | Me | Me | H | H | Me | H | - | |
| 2.179 | Me | Me | Me | H | H | Me | H | CH₂ | |
| 2.180 | Me | Me | Me | H | H | Me | H | O | |
| 2.181 | Et | H | Me | H | H | Me | H | - | |
| 2.182 | Et | H | Me | H | H | Me | H | CH₂ | |
| 2.183 | Et | H | Me | H | H | Me | H | O | |
| 2.184 | C(O)Me | H | H | H | H | Me | H | - | |
| 2.185 | C(O)Me | H | H | H | H | Me | H | CH₂ | |
| 2.186 | C(O)Me | H | H | H | H | Me | H | O | |
| 2.187 | C(O)Me | H | Me | H | H | Me | H | - | |
| 2.188 | C(O)Me | H | Me | H | H | Me | H | CH₂ | |
| 2.189 | C(O)Me | H | Me | H | H | Me | H | O | |
| 2.190 | C(O)Et | H | H | H | H | Me | H | - | |
| 2.191 | C(O)Et | H | H | H | H | Me | H | CH₂ | |
| 2.192 | C(O)Et | H | H | H | H | Me | H | O | |
| 2.193 | C(O)Et | H | Me | H | H | Me | H | - | |
| 2.194 | C(O)Et | H | Me | H | H | Me | H | CH₂ | |
| 2.195 | C(O)Et | H | Me | H | H | Me | H | O | |
| 2.196 | C(O)t-Bu | H | Me | H | H | Me | H | - | |
| 2.197 | C(O)t-Bu | H | Me | H | H | Me | H | CH₂ | |
| 2.198 | C(O)t-Bu | H | Me | H | H | Me | H | O | |
| 2.199 | C(O)CF₃ | H | H | H | H | Me | H | - | |
| 2.200 | C(O)CF₃ | H | H | H | H | Me | H | CH₂ | |
| 2.201 | C(O)CF₃ | H | H | H | H | Me | H | O | |
| 2.202 | C(O)CF₃ | H | Me | H | H | Me | H | - | |
| 2.203 | C(O)CF₃ | H | Me | H | H | Me | H | CH₂ | |
| 2.204 | C(O)CF₃ | H | Me | H | H | Me | H | O | |
| 2.205 | H | H | H | Me | H | H | H | O | |
| 2.206 | H | H | H | F | H | H | H | O | Ref. |
| 2.207 | H | H | H | H | H | t-Bu | H | - | |

| | | | | | | | | | |
|---|---|---|---|---|---|---|---|---|---|
| References to compounds (Ref.) in Table 2: Compound number 2.1: solid, melting point 181-182 °C Compound number 2.2: obtained as a waxy material Compound number 2.3: solid Compound number 2.5: solid, melting point 101-102 °C Compound number 2.25: solid Compound number 2.139: obtained as a waxy material Compound number 2.206: solid | | | | | | | | | |

**Table 3: Compounds of formula (Ib) (optically active compounds)**

| | | | | | | | | | |
|---|---|---|---|---|---|---|---|---|---|
| 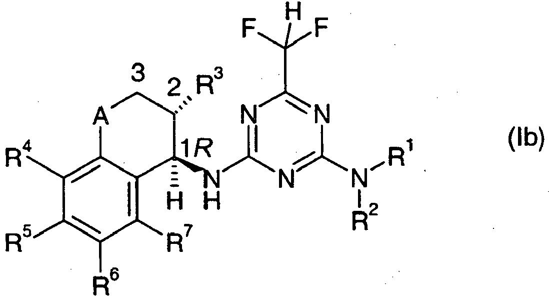 | | | | | | | | | |

| **Cpd** | **R¹** | **R²** | **R³** | **R⁴** | **R⁵** | **R⁶** | **R⁷** | **A** | **Ref.** |
|---|---|---|---|---|---|---|---|---|---|
| 3.1 | H | H | Me | H | H | Me | H | - | Ref. |
| 3.2 | H | H | Me | H | H | Me | H | CH₂ | |
| 3.3 | H | H | Me | H | H | Me | H | O | |
| 3.4 | H | H | Me | Me | H | Me | H | - | |
| 3.5 | H | H | Me | Me | H | Me | H | CH₂ | |
| 3.6 | H | H | Me | Me | H | Me | H | O | |
| 3.7 | H | H | Me | H | Me | Me | H | - | |
| 3.8 | H | H | Me | H | Me | Me | H | CH₂ | |
| 3.9 | H | H | Me | H | Me | Me | H | O | |
| 3.10 | H | H | Me | Me | Me | Me | H | - | |
| 3.11 | H | H | Me | Me | Me | Me | H | CH₂ | |
| 3.12 | H | H | Me | Me | Me | Me | H | O | |
| 3.13 | H | H | Me | H | H | Et | H | - | |
| 3.14 | H | H | Me | H | H | Et | H | CH₂ | |
| 3.15 | H | H | Me | H | H | Et | H | O | |
| 3.16 | H | H | Me | Me | H | Et | H | - | |
| 3.17 | H | H | Me | Me | H | Et | H | CH₂ | |
| 3.18 | H | H | Me | Me | H | Et | H | O | |
| 3.19 | H | H | Me | H | Me | Et | H | - | |
| 3.20 | H | H | Me | H | Me | Et | H | CH₂ | |
| 3.21 | H | H | Me | H | Me | Et | H | O | |
| 3.22 | H | H | Me | Me | Me | Et | H | - | |
| 3.23 | H | H | Me | Me | Me | Et | H | CH₂ | |
| 3.24 | H | H | Me | Me | Me | Et | H | O | |
| 3.25 | H | H | OMe | H | H | Me | H | - | |
| 3.26 | H | H | OMe | H | H | Me | H | CH₂ | |
| 3.27 | H | H | OMe | H | H | Me | H | O | |
| 3.28 | H | H | OMe | Me | H | Me | H | - | |
| 3.29 | H | H | OMe | Me | H | Me | H | CH₂ | |
| 3.30 | H | H | OMe | Me | H | Me | H | O | |
| 3.31 | H | H | Et | H | H | Me | H | - | Ref. |
| 3.32 | H | H | Et | H | H | Me | H | CH₂ | |
| 3.33 | H | H | Et | H | H | Me | H | O | |
| 3.34 | H | H | Et | Me | H | Me | H | - | |
| 3.35 | H | H | Et | Me | H | Me | H | CH₂ | |
| 3.36 | H | H | Et | Me | H | Me | H | O | |
| 3.37 | H | H | OMe | H | Me | Me | H | - | |
| 3.38 | H | H | OMe | H | Me | Me | H | CH₂ | |
| 3.39 | H | H | OMe | H | Me | Me | H | O | |
| 3.40 | H | H | OMe | Me | Me | Me | H | - | |
| 3.41 | H | H | OMe | Me | Me | Me | H | CH₂ | |
| 3.42 | H | H | OMe | Me | Me | Me | H | O | |
| 3.43 | H | H | Et | H | Me | Me | H | - | |
| 3.44 | H | H | Et | H | Me | Me | H | CH₂ | |
| 3.45 | H | H | Et | H | Me | Me | H | O | |
| 3.46 | H | H | Et | Me | Me | Me | H | - | |
| 3.47 | H | H | Et | Me | Me | Me | H | CH₂ | |
| 3.48 | H | H | Et | Me | Me | Me | H | O | |
| 3.49 | H | H | Me | H | H | i-Prop | H | - | |
| 3.50 | H | H | Me | H | H | i-Prop | H | CH₂ | |
| 3.51 | H | H | Me | H | H | i-Prop | H | O | |
| 3.52 | H | H | Et | H | H | i-Prop | H | - | |
| 3.53 | H | H | Et | H | H | i-Prop | H | CH₂ | |
| 3.54 | H | H | Et | H | H | i-Prop | H | O | |
| 3.55 | H | H | Me | H | H | OMe | H | - | |
| 3.56 | H | H | Me | H | H | OMe | H | CH₂ | |
| 3.57 | H | H | Me | H | H | OMe | H | O | |
| 3.58 | H | H | Et | H | H | OMe | H | - | |
| 3.59 | H | H | Et | H | H | OMe | H | CH₂ | |
| 3.60 | H | H | Et | H | H | OMe | H | O | |
| 3.61 | H | H | Me | H | H | OEt | H | - | |
| 3.62 | H | H | Me | H | H | OEt | H | CH₂ | |
| 3.63 | H | H | Me | H | H | OEt | H | O | |
| 3.64 | H | H | Et | H | H | OEt | H | - | |
| 3.65 | H | H | Et | H | H | OEt | H | CH₂ | |
| 3.66 | H | H | Et | H | H | OEt | H | O | |
| 3.67 | H | H | Me | H | H | CN | H | - | |
| 3.68 | H | H | Me | H | H | CN | H | CH₂ | |
| 3.69 | H | H | Me | H | H | CN | H | O | |
| 3.70 | H | H | Me | H | H | C≡CMe | H | - | |
| 3.71 | H | H | Me | H | H | C≡CMe | H | CH₂ | |
| 3.72 | H | H | Me | H | H | C≡CMe | H | O | |
| 3.73 | H | H | Me | H | H | C≡CH | H | - | |
| 3.74 | H | H | Me | H | H | C≡CH | H | CH₂ | |
| 3.75 | H | H | Me | H | H | C≡CH | H | O | |
| 3.76 | H | H | Me | H | H | F | H | - | |
| 3.77 | H | H | Me | H | H | F | H | CH₂ | |
| 3.78 | H | H | Me | H | H | F | H | O | |
| 3.79 | H | H | Me | H | H | Cl | H | - | |
| 3.80 | H | H | Me | H | H | Cl | H | CH₂ | |
| 3.81 | H | H | Me | H | H | Cl | H | O | |
| 3.82 | H | H | Me | H | H | Br | H | - | Ref. |
| 3.83 | H | H | Me | H | H | Br | H | CH₂ | |
| 3.84 | H | H | Me | H | H | Br | H | O | |
| 3.85 | H | H | Me | H | H | I | H | - | |
| 3.86 | H | H | Me | H | H | I | H | CH₂ | |
| 3.87 | H | H | Me | H | H | I | H | O | |
| 3.88 | H | H | OCH₂C≡CH | H | H | Me | H | - | |
| 3.89 | H | H | OCH₂C≡CH | H | H | Me | H | CH₂ | |
| 3.90 | H | H | OCH₂C≡CH | H | H | Me | H | O | |
| 3.91 | H | H | Me | H | H | OCH₂C≡CH | H | - | |
| 3.92 | H | H | Me | H | H | OCH₂C≡CH | H | CH₂ | |
| 3.93 | H | H | Me | H | H | OCH₂C≡CMe | H | O | |
| 3.94 | H | H | C≡CMe | H | H | Me | H | - | |
| 3.95 | H | H | C≡CMe | H | H | Me | H | CH₂ | |
| 3.96 | H | H | C≡CMe | H | H | Me | H | O | |
| 3.97 | Me | H | Me | H | H | Me | H | - | |
| 3.98 | Me | H | Me | H | H | Me | H | CH₂ | |
| 3.99 | Me | H | Me | H | H | Me | H | O | |
| 3.100 | Me | Me | Me | H | H | Me | H | - | |
| 3.101 | Me | Me | Me | H | H | Me | H | CH₂ | |
| 3.102 | Me | Me | Me | H | H | Me | H | O | |
| 3.103 | Et | H | Me | H | H | Me | H | - | |
| 3.104 | Et | H | Me | H | H | Me | H | CH₂ | |
| 3.105 | Et | H | Me | H | H | Me | H | O | |
| 3.106 | C(O)Me | H | Me | H | H | Me | H | - | Ref. |
| 3.107 | C(O)Me | H | Me | H | H | Me | H | CH₂ | |
| 3.108 | C(O)Me | H | Me | H | H | Me | H | O | |
| 3.109 | C(O)Et | H | Me | H | H | Me | H | - | Ref. |
| 3.110 | C(O)Et | H | Me | H | H | Me | H | CH₂ | |
| 3.111 | C(O)Et | H | Me | H | H | Me | H | O | |
| 3.112 | C(O)t-Bu | H | Me | H | H | Me | H | - | Ref. |
| 3.113 | C(O)t-Bu | H | Me | H | H | Me | H | CH₂ | |
| 3.114 | C(O)t-Bu | H | Me | H | H | Me | H | O | |
| 3.115 | C(O)CF₃ | H | Me | H | H | Me | H | - | |
| 3.116 | C(O)CF₃ | H | Me | H | H | Me | H | CH₂ | |
| 3.117 | C(O)CF₃ | H | Me | H | H | Me | H | O | |
| 3.118 | CHO | H | Me | H | H | Me | H | - | Ref. |

| | | | | | | | | | |
|---|---|---|---|---|---|---|---|---|---|
| References to compounds (Ref.) in Table 3: Compound number 3.1: solid, melting point 132 °C Compound number 3.31: solid, melting point 112 °C Compound number 3.82: solid Compound number 3.106: obtained as a waxy material Compound number 3.109: solid, melting point 66 °C Compound number 3.112: obtained as a waxy material Compound number 3.118: obtained as a waxy material | | | | | | | | | |

**Table 4: Compounds of formula (Ic) (optically active compounds)**

| | | | | | | | | | |
|---|---|---|---|---|---|---|---|---|---|
| 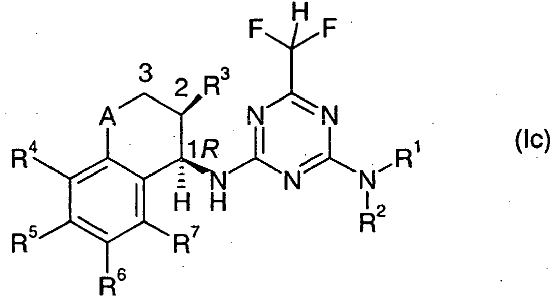 | | | | | | | | | |

| **Cpd** | **R¹** | **R²** | **R³** | **R⁴** | **R⁵** | **R⁶** | **R⁷** | **A** | **Ref.** |
|---|---|---|---|---|---|---|---|---|---|
| 4.1 | H | H | Me | H | H | Me | H | - | |
| 4.2 | H | H | Me | H | H | Me | H | CH₂ | |
| 4.3 | H | H | Me | H | H | Me | H | O | |
| 4.4 | H | H | Me | Me | H | Me | H | - | |
| 4.5 | H | H | Me | Me | H | Me | H | CH₂ | |
| 4.6 | H | H | Me | Me | H | Me | H | O | |
| 4.7 | H | H | Me | H | Me | Me | H | - | |
| 4.8 | H | H | Me | H | Me | Me | H | CH₂ | |
| 4.9 | H | H | Me | H | Me | Me | H | O | |
| 4.10 | H | H | Me | Me | Me | Me | H | - | |
| 4.11 | H | H | Me | Me | Me | Me | H | CH₂ | |
| 4.12 | H | H | Me | Me | Me | Me | H | O | |
| 4.13 | H | H | Me | H | H | Et | H | - | |
| 4.14 | H | H | Me | H | H | Et | H | CH₂ | |
| 4.15 | H | H | Me | H | H | Et | H | O | |
| 4.16 | H | H | Me | Me | H | Et | H | - | |
| 4.17 | H | H | Me | Me | H | Et | H | CH₂ | |
| 4.18 | H | H | Me | Me | H | Et | H | O | |
| 4.19 | H | H | Me | H | Me | Et | H | | |
| 4.20 | H | H | Me | H | Me | Et | H | CH₂ | |
| 4.21 | H | H | Me | H | Me | Et | H | O | |
| 4.22 | H | H | Me | Me | Me | Et | H | - | |
| 4.23 | H | H | Me | Me | Me | Et | H | CH₂ | |
| 4.24 | H | H | Me | Me | Me | Et | H | O | |
| 4.25 | H | H | OMe | H | H | Me | H | - | |
| 4.26 | H | H | OMe | H | H | Me | H | CH₂ | |
| 4.27 | H | H | OMe | H | H | Me | H | O | |
| 4.28 | H | H | OMe | Me | H | Me | H | - | |
| 4.29 | H | H | OMe | Me | H | Me | H | CH₂ | |
| 4.30 | H | H | OMe | Me | H | Me | H | O | |
| 4.31 | H | H | Et | H | H | Me | H | - | |
| 4.32 | H | H | Et | H | H | Me | H | CH₂ | |
| 4.33 | H | H | Et | H | H | Me | H | O | |
| 4.34 | H | H | Et | Me | H | Me | H | - | |
| 4.35 | H | H | Et | Me | H | Me | H | CH₂ | |
| 4.36 | H | H | Et | Me | H | Me | H | O | |
| 4.37 | H | H | OMe | H | Me | Me | H | - | |
| 4.38 | H | H | OMe | H | Me | Me | H | CH₂ | |
| 4.39 | H | H | OMe | H | Me | Me | H | O | |
| 4.40 | H | H | OMe | Me | Me | Me | H | - | |
| 4.41 | H | H | OMe | Me | Me | Me | H | CH₂ | |
| 4.42 | H | H | OMe | Me | Me | Me | H | O | |
| 4.43 | H | H | Et | H | Me | Me | H | - | |
| 4.44 | H | H | Et | H | Me | Me | H | CH₂ | |
| 4.45 | H | H | Et | H | Me | Me | H | O | |
| 4.46 | H | H | Et | Me | Me | Me | H | - | |
| 4.47 | H | H | Et | Me | Me | Me | H | CH₂ | |
| 4.48 | H | H | Et | Me | Me | Me | H | O | |
| 4.49 | H | H | Me | H | H | i-Prop | H | - | |
| 4.50 | H | H | Me | H | H | i-Prop | H | CH₂ | |
| 4.51 | H | H | Me | H | H | i-Prop | H | O | |
| 4.52 | H | H | Et | H | H | i-Prop | H | - | |
| 4.53 | H | H | Et | H | H | i-Prop | H | CH₂ | |
| 4.54 | H | H | Et | H | H | i-Prop | H | O | |
| 4.55 | H | H | Me | H | H | OMe | H | - | |
| 4.56 | H | H | Me | H | H | OMe | H | CH₂ | |
| 4.57 | H | H | Me | H | H | OMe | H | O | |
| 4.58 | H | H | Et | H | H | OMe | H | - | |
| 4.59 | H | H | Et | H | H | OMe | H | CH₂ | |
| 4.60 | H | H | Et | H | H | OMe | H | O | |
| 4.61 | H | H | Me | H | H | OEt | H | - | |
| 4.62 | H | H | Me | H | H | OEt | H | CH₂ | |
| 4.63 | H | H | Me | H | H | OEt | H | O | |
| 4.64 | H | H | Et | H | H | OEt | H | - | |
| 4.65 | H | H | Et | H | H | OEt | H | CH₂ | |
| 4.66 | H | H | Et | H | H | OEt | H | O | |
| 4.67 | H | H | Me | H | H | CN | H | - | |
| 4.68 | H | H | Me | H | H | CN | H | CH₂ | |
| 4.69 | H | H | Me | H | H | CN | H | O | |
| 4.70 | H | H | Me | H | H | C≡CMe | H | - | |
| 4.71 | H | H | Me | H | H | C≡CMe | H | CH₂ | |
| 4.72 | H | H | Me | H | H | C≡CMe | H | O | |
| 4.73 | H | H | Me | H | H | C≡CH | H | - | |
| 4.74 | H | H | Me | H | H | C≡CH | H | CH₂ | |
| 4.75 | H | H | Me | H | H | C≡CH | H | O | |
| 4.76 | H | H | Me | H | H | F | H | - | |
| 4.77 | H | H | Me | H | H | F | H | CH₂ | |
| 4.78 | H | H | Me | H | H | F | H | O | |
| 4.79 | H | H | Me | H | H | Cl | H | - | |
| 4.80 | H | H | Me | H | H | Cl | H | CH₂ | |
| 4.81 | H | H | Me | H | H | Cl | H | O | |
| 4.82 | H | H | Me | H | H | Br | H | - | |
| 4.83 | H | H | Me | H | H | Br | H | CH₂ | |
| 4.84 | H | H | Me | H | H | Br | H | O | |
| 4.85 | H | H | Me | H | H | I | H | - | |
| 4.86 | H | H | Me | H | H | I | H | CH₂ | |
| 4.87 | H | H | Me | H | H | I | H | O | |
| 4.88 | H | H | OCH₂C≡CH | H | H | Me | H | - | |
| 4.89 | H | H | OCH₂C≡CH | H | H | Me | H | CH₂ | |
| 4.90 | H | H | OCH₂C≡CH | H | H | Me | H | O | |
| 4.91 | H | H | Me | H | H | OCH₂C≡CH | H | - | |
| 4.92 | H | H | Me | H | H | OCH₂C≡CH | H | CH₂ | |
| 4.93 | H | H | Me | H | H | OCH₂C≡CH | H | O | |
| 4.94 | H | H | C≡CMe | H | H | Me | H | - | |
| 4.95 | H | H | C≡CMe | H | H | Me | H | CH₂ | |
| 4.96 | H | H | C≡CMe | H | H | Me | H | O | |
| 4.97 | Me | H | Me | H | H | Me | H | - | |
| 4.98 | Me | H | Me | H | H | Me | H | CH₂ | |
| 4.99 | Me | H | Me | H | H | Me | H | O | |
| 4.100 | Me | Me | Me | H | H | Me | H | - | |
| 4.101 | Me | Me | Me | H | H | Me | H | CH₂ | |

| **Cpd** | **R¹** | **R²** | **R³** | **R⁴** | **R⁶** | **R⁶** | **R⁷** | **A** | **Ref.** |
|---|---|---|---|---|---|---|---|---|---|
| 4.102 | Me | Me | Me | H | H | Me | H | O | |
| 4.103 | Et | H | Me | H | H | Me | H | - | |
| 4.104 | Et | H | Me | H | H | Me | H | CH₂ | |
| 4.105 | Et | H | Me | H | H | Me | H | O | |
| 4.106 | C(O)Me | H | Me | H | H | Me | H | - | |
| 4.107 | C(O)Me | H | Me | H | H | Me | H | CH₂ | |
| 4.108 | C(O)Me | H | Me | H | H | Me | H | O | |
| 4.109 | C(O)Et | H | Me | H | H | Me | H | - | |
| 4.110 | C(O)Et | H | Me | H | H | Me | H | CH₂ | |
| 4.111 | C(O)Et | H | Me | H | H | Me | H | O | |
| 4.112 | C(O)t-Bu | H | Me | H | H | Me | H | - | |
| 4.113 | C(O)t-Bu | H | Me | H | H | Me | H | CH₂ | |
| 4.114 | C(O)t-Bu | H | Me | H | H | Me | H | O | |
| 4.115 | C(O)CF₃ | H | Me | H | H | Me | H | - | |
| 4.116 | C(O)CF₃ | H | Me | H | H | Me | H | CH₂ | |
| 4.117 | C(O)CF₃ | H | Me | H | H | Me | H | O | |
| 4.118 | CHO | H | Me | H | H | Me | H | - | |

According to a further feature of the present invention, there is provided the use as a herbicide or plant growth regulator characterised in that the compound of formula (I) or a salt thereof is applied in an effective amount for the control of harmful plants (weeds) or for regulating the growth of plants at a plant, locus. For this purpose, the said compound is normally used in the form of a herbicidal composition (i.e. in association with compatible diluents or carriers and/or surface active agents suitable for use in herbicidal compositions), for example as hereinafter described.

By application to the 'plant locus' is meant application, for example to plants, plant seeds, plant parts or the area where the plants are growing or to be grown, or particularly application to the plant growing medium, such as soil, as well as to the seeds, emerging seedlings, roots, stems, leaves or other plant parts.

The compounds of the formula (I) and their salts, all termed hereinbelow as compounds of formula (I), have an excellent herbicidal activity against a broad range of economically important monocotyledonous and dicotyledonous harmful plants. The compounds of formula (I) also act efficiently on perennial weeds which produce shoots from rhizomes, root stocks or other perennial organs and which are difficult to control. In this context, the substances can be applied pre-planting, pre-emergence or post-emergence.

Specifically, examples may be mentioned of some representatives of the monocotyledonous and dicotyledonous weed flora which can be controlled by the compounds of formula (I), without the enumeration being a restriction to certain species.

Amongst the monocotyledonous weed species, those on which the active substances act efficiently are, for example, Agrostis, Alopecurus, Apera, Avena, Brachicaria, Bromus, Dactyloctenium, Digitaria, Echinochloa, Eleocharis, Eleusine, Festuca, Fimbristylis, lschaemum, Lolium, Monochoria, Panicum, Paspalum, Phalaris, Phleum, Poa, Sagittaria, Scirpus, Setaria, Sphenoclea and Cyperus species from the annual group and, amongst the perennial species, Agropyron, Cynodon, Imperata and Sorghum and also perennial Cyperus species.

In the case of dicotyledonous weed species, the spectrum of action extends to species such as, for example, Galium, Viola, Veronica, Lamium, Stellaria, Amaranthus, Sinapis, Ipomoea, Matricaria, Abutilon and Sida amongst the annuals and Convolvulus, Cirsium, Rumex and Artemisia in the case of the perennial weeds. Herbicidal action is also achieved in the case of dicotyledonous harmful plants such as Ambrosia, Anthemis, Carduus, Centaurea, Chenopodium, Cirsium, Convolvulus, Datura, Emex, Galeopsis, Galinsoga, Kochia, Lepidium, Lindernia, Papaver, Portlaca, Polygonum, Ranunculus, Rorippa, Rotala, Seneceio, Sesbania, Solanum, Sonchus, Taraxacum, Trifolium, Urtica and Xanthium.

Harmful plants occurring under the specific cultivation conditions of rice, such as, for example, Sagittaria, Alisma, Eleocharis, Scirpus and Cyperus, are also well controlled by the active substances according to the invention.

If the compounds according to the invention are applied to the soil surface before germination (pre-emergence of the weeds), then the weed seedlings are either prevented completely from emerging or the weeds grow until they have reached the cotyledon stage, but then their growth stops and they finally die completely after three to four weeks have elapsed.

When the active substances are applied post-emergence to the green parts of the plants, growth stops equally drastically a very short time after treatment and the weed plants remain at the stage of growth at the time of application, or they die completely after a certain time, so that in this manner competition by the weeds, which is harmful to the crop plants, is eliminated at a very early stage and in a sustained manner.

Although the compounds according to the invention have an excellent herbicidal activity against mono- and dicotyledonous weeds, some crop plants of economically important crops such as, for example, wheat, barley, rye, triticale, rice, maize, sugar beet, oil-seed rape, cotton or soybeans (particularly wheat, barley, rice or maize) are damaged only to an insignificant extent or not at all, if an appropriate dosage is applied. For these reasons, the present compounds are in some cases suitable for the selective control of undesired vegetation in stands of agriculturally useful plants or in stands of ornamental plants.

The activity allows to employ the compounds as effective herbicidal active ingredients pre- and post-emergence for controlling broad-leaved weeds and grass weeds at relatively low dosage as a selective herbicide in some crops (preferably pre-emergence of the weeds in many cases). Alternatively the compounds can be used effectively at some higher dosage for the control of a broad range of dicotyledonous weeds and monocotyledonous weeds in plantation crops and on uncultivated land and, by means of specific application techniques, also for inter-row treatment in agricultural row crops such as maize, cotton and the like.

The compositions according to the invention can be used to selectively control annual and perennial harmful plants in plantation crops such as oil palm, coconut palm, nuts (for example almonds, hazelnuts, walnuts, macadamia), berries, citrus (for example orange, lemon, mandarin), bananas, sugar cane, christmas tree, eucalyptus, (India-)rubber tree, pineapples, cotton, coffee, cocoa and the like, as well as in fruit production (e.g. pome fruit such as apple, pear, cherry, mango, kiwi) and viticulture. Equally, the combinations according to the invention can be employed in arable crop production using the no-till, or zero-till, method or for treatment after harvest ("chemical fallow").

Another object of the invention is thus the selective weed control in plantation crops by applying the compounds according to the invention as herbicides.

Alternatively, they can be used as very effective herbicides in a non-selective manner on paths, open spaces and industrial sites and the like to keep these areas free from undesirable vegetation.

The herbicidal compositions according to the invention are distinguished by a long-term herbicidal action with a rapid onset.

In addition, the substances according to the invention have outstanding growth-regulatory properties in crop plants. They engage in the plants' metabolism in a regulatory fashion and can thus be employed for influencing plant constituents in a targeted fashion and for facilitating harvesting, such as, for example, by triggering desiccation and stunted growth. Moreover, they are also suitable for generally controlling and inhibiting undesired vegetative growth without simultaneously killing the plants. Inhibiting vegetative growth plays an important role in many monocotyledonous and dicotyledonous crops since lodging can be reduced, or prevented completely, hereby.

The compounds of formula (I) are very active herbicides from the class of 2,4-diamino-1,3,5-triazines substituted at the triazine ring with a benzocondensated (hetero)cycloalkyl radical and can be used for the control of a range of harmful weeds. The compounds have an effect on the biosynthesis of cellulose in plants and thus on the formation of plant cell walls. They possess surprisingly more potent herbicidal efficacy than structurally close compounds known from said class.

Due to their herbicidal and plant-growth regulatory properties, the compounds of formula (I) can also be employed for controlling harmful plants in crops of known genetically modified plants, or genetically modified plants yet to be developed. As a rule, the transgenic plants are distinguished by particular advantageous properties, for example by resistances to certain pesticides, mainly certain herbicides, resistances to plant diseases, or pathogens of plant diseases, such as certain insects or microorganisms such as fungi, bacteria or viruses. Other particular properties relate, for example, to the harvested material with regard to quantity, quality, storage properties, composition and specific constituents. Thus, transgenic plants are known where the starch content is increased or the starch quality is altered or those where the harvested material has a different fatty acid spectrum.

The compounds of formula (I) are preferably employed in economically important transgenic crops of useful plants and ornamentals, for example cereals such as wheat, barley, rye, oats, sorghum and millet, rice, cassava and maize, or else crops of sugar beet, cotton, soya, oil seed rape, potatoes, tomatoes, peas and other vegetables.

The compounds of formula (I) can preferably be employed as herbicides in crops of useful plants which are resistant to the phytotoxic effects of the herbicides or have been rendered thus by means of genetic engineering.

Traditional ways of generating novel plants which have modified characteristics in comparison with existing plants consist, for example, in traditional breeding methods and the generation of mutants. However, it is also possible to generate novel plants with altered characteristics with the aid of genetic engineering methods (see, for example, EP-A-0221044, EP-A-0131624). For example, several cases have been described of
- genetic engineering modifications of crop plants with the purpose of modifying the starch synthesized in the plants (for example WO 92/11376, WO 92/14827, WO 91/19806),
- transgenic crop plants which are resistant to certain herbicides of the glufosinate type (cf., for example, EP-A-0242236, EP-A-242246) or the glyphosate type (WO 92/00377) or the sulfonylurea type (EP-A-0257993, US-A-5013659),
- transgenic crop plants, for example cotton, which are capable of producing Bacillus thuringiensis toxins (Bt toxins) which make the plants resistant to specific pests (EP-A-0142924, EP-A-0193259),
- transgenic crop plants whose fatty acid spectrum is modified (WO 91/13972).

A large number of techniques in molecular biology by means of which novel transgenic plants with altered characteristics can be generated are known in principle; see, for example, Sambrook et al., 1989, Molecular Cloning, A Laboratory Manual, 2nd Ed., Cold Spring Harbor Laboratory Press, Cold Spring Harbor, NY; or Winnacker "Gene und Klone" [Genes and Clones], VCH Weinheim 2nd Edition 1996, or Christou, "Trends in Plant Science" 1 (1996) 423-431).

In order to perform such genetic engineering manipulations, nucleic acid molecules may be introduced into plasmids which allow mutagenesis or a sequence change by means of recombination of DNA sequences. It is possible, for example, with the aid of the abovementioned standard methods to perform base exchanges, to remove subsequences or to add natural or synthetic sequences. To connect the DNA fragments to each other, adaptors or linkers may be attached to the fragments.

For example, plant cells with a reduced activity of a gene product can be generated by expressing at least one corresponding antisense RNA, a sense RNA to achieve a cosuppressory effect or by expressing at least one ribozyme of suitable construction which specifically cleaves transcripts of the abovementioned gene product.

To this end it is possible to make use of, on the one hand, DNA molecules which encompass the entire coding sequence of a gene product inclusive of any flanking sequences which may be present, on the other hand DNA molecules which only encompass parts of the coding sequence, but these parts must be long enough in order to effect, in the cells, an antisense effect. Use may also be made of DNA sequences which show a high degree of homology to the coding sequences of a gene product, but which are not completely identical.

When nucleic acid molecules are expressed in plants, the protein which has been synthesized may be located in any desired compartment of the plant cell. However, to achieve localization in a particular compartment, it is possible, for example, to link the coding region with DNA sequences which guarantee localization in a particular compartment. Such sequences are known to the skilled worker (see, for example, Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

The transgenic plant cells may be regenerated by known techniques to give complete plants. In principle, the transgenic plants can be plants of any desired plant species, that is to say monocotyledonous and also dicotyledonous plants.

This allows transgenic plants to be obtained which exhibit altered characteristics by means of overexpression, suppression or inhibition of homologous (= natural) genes or gene sequences or by means of expression of heterologous (= foreign) genes or gene sequences.

The compounds of formula (I) can preferably be employed in transgenic crops which are resistant to herbicides from the group of the sulfonylureas, imidazolinones, glufosinate-ammonium or glyphosate-isopropylammonium or inhibitors of HPPD (*p-*Hydroxyphenyl pyruvate dioxygenase inhibitor, which ultimately affect carotenoid biosynthesis in plants) and analogous active substances.

When the compounds of formula (I) are used in transgenic crops, effects other than the herbicidal effects to be observed in other crops are frequently found which are specific for application in the particular transgenic crop, for example an altered or specifically widened weed spectrum which can be controlled, altered application rates which may be employed for application, preferably good combining ability with the herbicides to which the transgenic crop is resistant, and an effect on growth and yield of the transgenic crop plants.

The invention therefore also relates to the use of the compounds of formula (I) as herbicides for controlling harmful plants in transgenic crop plants.

The invention thus also relates to a method of controlling undesirable vegetation which comprises applying one or more herbicides of formula (I) together with one or more other pesticides, preferably other herbicides, and optionally formulation auxiliaries such as a surfactant, to the harmful plants, parts of these plants or the area under cultivation.

The use according to the invention for controlling harmful plants or for regulating the growth of plants also includes the case where the compounds of formula (I) are only formed in the plant or the soil from a precursor ("prodrug") after its application to the plant.

The compounds of formula (I) can be employed in the conventional preparations as wettable powders, emulsifiable concentrates, sprayable solutions, dusts or granules. The invention therefore also relates to herbicidal and plant-growth-regulating compositions which comprise compounds of formula (I).

According to a further feature of the present invention, there is provided a herbicidal or plant growth regulating composition comprising an effective amount of a compound of formula (I) as defined above or an agriculturally acceptable salt thereof, in association with, and preferably homogeneously dispersed in, one or more compatible agriculturally- acceptable diluents or carriers arid/or surface active agents [i.e. diluents or carriers and/or surface active agents of the type generally accepted in the art as being suitable for use in herbicidal compositions and which are compatible with compounds of the invention]. The term "homogeneously dispersed" is used to include compositions in which the compounds of formula (I) are dissolved in other components. The term "herbicidal compositions" is used in a broad sense to include not only compositions which are ready for use as herbicides but also concentrates which must be diluted before use (including tank mixtures). The compounds of formula (I) can be formulated in various ways, depending on the prevailing biological and/or chemico-physical parameters. Examples of possible formulations which are suitable are: wettable powders (WP), water-soluble powders (SP), water-soluble concentrates, emulsifiable concentrates (EC), emulsions (EW) such as oil-in-water and water-in-oil emulsions, sprayable solutions, suspension concentrates (SC), dispersions on an oil or water basis, solutions which are miscible with oil, capsule suspensions (CS), dusts (DP), seed-dressing products, granules for broadcasting and soil application, granules (GR) in the form of microgranules, spray granules, coated granules and adsorption granules, water-dispersible granules (WG), water-soluble granules (SG), ULV formulations, microcapsules and waxes.

These individual formulation types are known in principle and described, for example, in: Winnacker-Küchler, "Chemische Technologie" [Chemical Technology], Volume 7, C. Hanser Verlag, Munich, 4th Edition 1986; Wade van Valkenburg, "Pesticide Formulations", Marcel Dekker, N.Y., 1973; K. Martens, "Spray Drying Handbook", 3rd Ed. 1979, G. Goodwin Ltd. London.

The necessary formulation auxiliaries such as inert materials, surfactants, solvents and other additives are also known and described, for example, in: Watkins, "Handbook of Insecticide Dust Diluents and Carriers", 2nd Ed., Darland Books, Caldwell N.J.; H.v. Olphen, "Introduction to Clay Colloid Chemistry", 2nd Ed., J. Wiley & Sons, N.Y.; C. Marsden, "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte" [Surface-active ethylene oxide adducts], Wiss. Verlagsgesell., Stuttgart 1976; Winnacker-Küchler, "Chemische Technologie" [Chemical Technology], Volume 7, C. Hanser Verlag, Munich, 4th Ed. 1986.

Based on these formulations, it is also possible to prepare combinations with other pesticidally active substances such as, for example, insecticides, acaricides, herbicides, fungicides, and with safeners, fertilizers and/or growth regulators, for example in the form of a readymix or a tank mix.

Wettable powders are preparations which are uniformly dispersible in water and which, besides the compounds of formula (I), also comprise ionic and/or nonionic surfactants (wetters, dispersants), for example, polyoxyethylated alkylphenols, polyoxyethylated fatty alcohols, polyoxyethylated fatty amines, fatty alcohol polyglycol ether sulfates, alkanesulfonates or alkylbenzenesulfonates, sodium lignosulfonate, sodium 2,2'-dinaphthylmethane-6,6'-disulfonate, sodium dibutylnaphthalenesulfonate or else sodium oleoylmethyltaurinate, in addition to a diluent or inert substance. To prepare the wettable powders, the compounds of formula (I) are, for example, ground finely in conventional apparatuses such as hammer mills, blower mills and air-jet mills and mixed with the formulation auxiliaries, either concomitantly or thereafter.

Emulsifiable concentrates are prepared, for example, by dissolving the compounds of formula (I) in an organic solvent, for example butanol, cyclohexanone, dimethylformamide, xylene or else higher-boiling aromatics or hydrocarbons or mixtures of these, with addition of one or more ionic and/or nonionic surfactants (emulsifiers). Emulsifiers which can be used are, for example: calcium salts of alkylarylsulfonic acids, such as calcium dodecylbenzenesulfonate or nonionic emulsifiers, such as fatty acid polyglycol esters, alkylaryl polyglycol ethers, fatty alcohol polyglycol ethers, propylene oxide/ethylene oxide condensates, alkyl polyethers, sorbitan esters such as sorbitan fatty acid esters or polyoxyethylene sorbitan esters such as polyoxyethylene sorbitan fatty acid esters.

Dusts are obtained by grinding the active substance with finely divided solid substances, for example talc or natural clays, such as kaolin, bentonite or pyrophyllite, or diatomaceous earth.

Suspension concentrates may be water- or oil-based. They can be prepared, for example, by wet grinding by means of commercially available bead mills, if appropriate with addition of surfactants, as they have already been mentioned above for example in the case of the other formulation types.

Emulsions, for example oil-in-water emulsions (EW), can be prepared for example by means of stirrers, colloid mills and/or static mixtures using aqueous organic solvents and, if appropriate, surfactants as they have already been mentioned above for example in the case of the other formulation types.

Granules can be prepared either by spraying the compounds of formula (I) onto adsorptive, granulated inert material or by applying active substance concentrates onto the surface of carriers such as sand, kaolinites or of granulated inert material, by means of binders, for example polyvinyl alcohol, sodium polyacrylate or alternatively mineral oils. Suitable active substances can also be granulated in the manner which is conventional for the production of fertilizer granules, if desired in a mixture with fertilizers.

Water-dispersible granules are prepared, as a rule, by the customary processes such as spray-drying, fluidized-bed granulation, disk granulation, mixing in highspeed mixers and extrusion without solid inert material. To prepare disk, fluidized-bed, extruder and spray granules, see, for example, processes in "Spray-Drying Handbook" 3rd ed. 1979, G. Goodwin Ltd., London; J.E. Browning, "Agglomeration", Chemical and Engineering 1967, pages 147 et seq.; "Perry's Chemical Engineer's Handbook", 5th Ed., McGraw-Hill, New York 1973, p. 8-57.

For further details on the formulation of crop protection products, see, for example, G.C. Klingman, "Weed Control as a Science", John Wiley and Sons, Inc., New York, 1961, pages 81-96 and J.D. Freyer, S.A. Evans, "Weed Control Handbook", 5th Ed., Blackwell Scientific Publications, Oxford, 1968, pages 101-103.

As a rule, the agrochemical preparations comprise 0.1 to 99% by weight, in particular 0.1 to 95% by weight, of compounds of formula (I).

The concentration of compounds of formula (I) in wettable powders is, for example, approximately 10 to 90% by weight, the remainder to 100% by weight being composed of customary formulation components. In the case of emulsifiable concentrates, the concentration of compounds of formula (I) can amount to approximately 1 to 90, preferably 5 to 80% by weight. Formulations in the form of dusts usually comprise 1 to 30% by weight of compounds of formula (I), preferably in most cases 5 to 20% by weight of compounds of formula (I), while sprayable solutions comprise approximately 0.05 to 80, preferably 2 to 50% by weight of compounds of formula (I). In the case of water-dispersible granules, the content of compounds of formula (I) depends partly on whether the compounds of formula (I) are in liquid or solid form and on which granulation auxiliaries, fillers and the like are being used. The water-dispersible granules, for example, comprise between 1 and 95% by weight of active substance, preferably between 10 and 80% by weight.

In addition, the formulations of compounds of formula (I) mentioned comprise, if appropriate, the adhesives, wetters, dispersants, emulsifiers, penetrants, preservatives, antifreeze agents, solvents, fillers, carriers, colorants, antifoams, evaporation inhibitors, pH regulators and viscosity regulators which are conventional in each case.

The compounds of the formula (I) or their salts can be employed as such or in the form of their preparations (formulations) as combinations with other pesticidally active substances, such as, for example, insecticides, acaricides, nematicides, herbicides, fungicides, safeners, fertilizers and/or growth regulators, for example as a premix or as tank mixes.

Components which may be employed for the active substances according to the invention in mixed formulations or in tank mix can be from entirely different structural classes and entirely different mechanisms of action, for example, active compounds which are based on an inhibition of, for example, acetolactate synthase, acetyl-coenzyme A carboxylase, PS I, PS II, HPPDO, phytoene desaturase, protoporphyrinogen oxidase, glutamine synthetase, cellulose biosynthesis, 5-enolpyruvylshikimate-3-phosphate synthetase. Such compounds, and also other compounds which can be employed, whose mechanism of action is to a degree unknown or different, are described, for example, in the handbook "The Pesticide Manual", 14th Edition 2006 or former editions, The British Crop Protection Council (abbreviation herein "PM") and the corresponding "e-Pesticide Manual", Version 4 (2006) or former versions, or else trade names and common names which are referred to in the "Compendium of Pesticide Common Names" (searchable via the Internet) and in literature quoted therein. The herbicides and plant growth regulators mentioned hereinbelow by way of example are in each case referred to by their standardized common active compound name according to the "International Organization for Standardization" (ISO), or by the chemical name or the code number: acetochlor, acibenzolar-S-methyl, acifluorfen(-sodium), aclonifen, AD-67, AKH 7088, i.e. [[[1-[5-[2-chloro-4-(trifluoromethyl)phenoxy]-2-nitrophenyl]-2-methoxyethylidene]amino]oxy]acetic acid and its methyl ester, alachlor, alloxydim(-sodium), ametryn, amicarbazone, amidochlor, amidosulfuron, aminopyralid, amitrol, AMS, i.e. ammonium sulfamate, ancimidol, anilofos, asulam, atrazine, aviglycine, azafenidin, azimsulfuron (DPX-A8947), aziprotryn, barban, BAS 516 H, i.e. 5-fluoro-2-phenyl-4H-3,1-benzoxazin-4-one, beflubutamid, benazolin(-ethyl), bencarbzone, benfluralin, benfuresate, benoxacor, bensulfuron(-methyl), bensulide, bentazone(-sodium), benzfendizone, benzobicyclone, benzofenap, benzofluor, benzoylprop(-ethyl), benzthiazuron, bialaphos (bilanafos), bifenox, bispyribac(-sodium), borax, bromacil, bromobutide, bromofenoxim, bromoxynil, bromuron, buminafos, busoxinone, butachlor, butafenacil, butamifos, butenachlor, buthidazole, butralin, butroxydim, butylate, cafenstrole (CH-900), carbetamide, carfentrazone(-ethyl), caloxydim, CDAA, i.e. 2-chloro-N,N-di-2-propenylacetamide, CDEC, i.e. 2-chloroallyl diethyldithiocarbamate, chlomethoxyfen, chloramben, chlorazifop-butyl, chlorbromuron, chlorbufam, chlorfenac, chlorfenprop, chlorfurecol(-methyl), chlorflurenol(-methyl), chloridazon, chlorimuron(-ethyl), chlormequat(-chloride), chlornitrofen, chlorotoluron, chloroxuron, chlorpropham, chlorsulfuron, chlorthal-dimethyl, chlorthiamid, chlortoluron, cinidon(-methyl or -ethyl), cinmethylin, cinosulfuron, clethodim, clefoxydim, clodinafop and its ester derivatives (for example clodinafop-propargyl), clofencet, clomazone, clomeprop, cloprop, cloproxydim, clopyralid, clopyrasulfuron(-methyl), cloquintocet(-mexyl), cloransulam(-methyl), cumyluron (JC 940), cyanamide, cyanazine, cycloate, cyclosulfamuron (AC 104), cycloxydim, cycluron, cyhalofop and its ester derivatives (for example butyl ester, DEH-112), cyperquat, cyprazine, cyprazole, cyprosulfamide, daimuron, 2,4-D, dalapon, daminozide, dazomet, n-decanol, dazomet, desmedipham, desmetryn, di-allate, dicamba, dichlobenil, dichlormid, dichlorprop(-P), diclofop and its esters such as diclofop-methyl, diclofop-P-(methyl), diclosulam, diethatyl(-ethyl), difenoxuron, difenzoquat(-metilsulfate), diflufenican, diflufenzopyr, dimefuron, dimepiperate, dimethachlor, dimethametryn, dimethazone, dimethenamid (SAN-582H), dimethenamid-P, dimethylarsinic acid, dimethipin, dimexyflam, dimetrasulfuron, dinitramine, dinoseb, dinoterb, diphenamid, dipropetryn, diquatsalts, dithiopyr, diuron, DNOC, eglinazine-ethyl, EL 77, i.e. 5-cyano-1-(1,1-dimethylethyl)-N-methyl-1H-pyrazole-4-carboxamide, endothal, epoprodan, EPTC, esprocarb, ethalfluralin, ethametsulfuron-methyl, ethephon, ethidimuron, ethiozin, ethofumesate, ethoxyfen and its esters (for example ethyl ester, HC-252), ethoxysulfuron, etobenzanid (HW 52), F5231, i.e. N-[2-chloro-4-fluoro-5-[4-(3-fluoropropyl)-4,5-dihydro-5-oxo-1H-tetrazol-1-yl]-phenyl]ethanesulfonamide, fenchlorazole(-ethyl), fenclorine, fenoprop, fenoxan, fenoxaprop and fenoxaprop-P and their esters, for example fenoxaprop-P-ethyl and fenoxaprop-ethyl, fenoxydim, fentrazamide, ferrous sulfate, fenuron, flamprop(-methyl or -isopropyl or -isopropyl-L), flamprop-M(-methyl or -isopropyl), flazasulfuron, florasulam, fluazifop and fluazifop-P and their esters, for example fluazifop-butyl and fluazifop-P-butyl, fluazolate, flucarbazone(-sodium), flucetosulfuron, fluchloralin, flufenacet (FOE 5043, also known as fluthiamide), flufenpyr(-ethyl), flumetralin, flumetsulam, flumeturon, flumiclorac(-pentyl), flumioxazin (S-482), flumipropyn, fluometuron, fluorochloridone, fluorodifen, fluoroglycofen(-ethyl), flupoxam (KNW-739), flupropacil (UBIC-4243), flupropanoate, fluproanate, flupyrsulfuron(-methyl, or -sodium), flurazole, flurenol(-butyl), fluridone, flurochloridone, fluroxypyr(-meptyl), flurprimidol, flurtamone, fluthiacet(-methyl), fluthiamide (also known as flufenacet), fomesafen, foramsulfuron, fosamine, furilazole (MON 13900), furyloxyfen, gibberillic acid, glufosinate (-ammonium), glyphosate(-isopropylammonium), halosafen, halosulfuron(-methyl), haloxyfop and its esters, haloxyfop-P (= R-haloxyfop) and its esters, HC-252 (diphenyl ether), hexazinone, imazamethabenz(-methyl), imazamethapyr, imazamox, imazapic, imazapyr, imazaquin and salts such as the ammonium salts, imazethamethapyr, imazethapyr, imazosulfuron, inabenfide, indanofan, indol-3-acetic acid, 4-indol-3-yl-butyric acid, iodosulfuron(-methyl)(-sodium), ioxynil, isocarbamid, isopropalin, isoproturon, isouron, isoxaben, isoxachlortole, isoxadifen(-ethyl), isoxaflutole, isoxapyrifop, karbutilate, lactofen, lenacil, linuron, MCPA, MCPA-thioethyl, MCPB, mecoprop(-P), mefenacet, mefenpyr(-diethyl), mefluidid, mepiquat(-chloride), mesosulfuron(-methyl), mesotrione, metam, metamifop, metamitron, metazachlor, methabenzthiazuron, methazole, methoxyphenone, methylarsonic acid, methylcyclopropene, methyldymron, methylisothiacyanate, methabenzthiazuron, metobromuron, (S-)metolachlor, metosulam (XRD 511), metoxuron, metribuzin, metsulfuron-methyl, MK-616, molinate, monalide, monocarbamide dihydrogensulfate, monolinuron, monuron, MT 128, i.e. 6-chloro-N-(3-chloro-2-propenyl)-5-methyl-N-phenyl-3-pyridazinamine, MT 5950, i.e. N-[3-chloro-4-(1-methylethyl)-phenyl]-2-methylpentanamide, naproanilide, napropamide, naptalam, NC 310, i.e. 4-(2,4-dichlorobenzoyl)-1-methyl-5-benzyloxypyrazole, neburon, nicosulfuron, nipyraclofen, nitralin, nitrofen, nitrofluorfen, nitrophenolate mixture, nonanoic acid norflurazon, orbencarb, orthosulfamuron, oxabetrinil, oryzalin, oxadiargyl (RP-020630), oxadiazone, oxasulfuron, oxaziclomefone, oxyfluorfen, paclobutrazol, paraquat(-dichloride), pebulate, pelargonic acid, pendimethalin, penoxulam, pentachlorphenol, pentanochlor, pentoxazone, perfluidone, pethoxamid, phenisopham, phenmedipham, picloram, picolinafen, pinoxaden, piperophos, piributicarb, pirifenop-butyl, pretilachlor, primisulfuron(-methyl), probenazole, procarbazone(-sodium), procyazine, prodiamine, profluazole, profluralin, profoxydim, prohexadione(-calcium), prohydrojasmon, proglinazine(-ethyl), prometon, prometryn, propachlor, propanil, propaquizafop, propazine, propham, propisochlor, propoxycarbazone(-sodium), n-propyl-dihydrojasmonate, propyzamide, prosulfalin, prosulfocarb, prosulfuron (CGA-152005), prynachlor, pyraclonil, pyraflufen(-ethyl), pyrasulfutole, pyrazolynate, pyrazon, pyrazosulfuron(-ethyl), pyrazoxyfen, pyribenzoxim, pyributicarb, pyridafol, pyridate, pyriftalid, pyrimidobac(-methyl), pyrimisulfan, pyrithiobac(-sodium) (KIH-2031), pyroxasulfone, pyroxofop and its esters (for example propargyl ester), proxulam, quinclorac, quinmerac, quinoclamine, quinofop and its ester derivatives, quizalofop and quizalofop-P and their ester derivatives, for example quizalofop-ethyl, quizalofop-P-tefuryl and -ethyl, renriduron, rimsulfuron (DPX-E 9636), S 275, i.e. 2-[4-chloro-2-fluoro-5-(2-propynyloxy)phenyl]-4,5,6,7-tetrahydro-2H-indazole, secbumeton, sethoxydim, siduron, simazine, simetryn, sintofen, SN 106279, i.e. 2-[[7-[2-chloro-4-(trifluoromethyl)phenoxy]-2-naphthalenyl]oxy]-propanoic acid and its methyl ester, sulcotrione, sulfentrazone (FMC-97285, F-6285), sulfazuron, sulfometuron(-methyl), sulfosate (ICI-A0224), sulfosulfuron, TCA, tebutam (GCP-5544), tebuthiuron, tecnacene, tembotrione, tefuryltrione, tepraloxydim, terbacil, terbucarb, terbuchlor, terbumeton, terbuthylazine, terbutryn, TFH 450, i.e. N,N-diethyl-3-[(2-ethyl-6-methylphenyl)sulfonyl]-1H-1,2,4-triazole-1-carboxamide, thenylchlor (NSK-850), thiafluamide, thiazafluron, thiazopyr (Mon-13200), thidiazimin (SN-24085), thidiazuron, thidiazuron, thiencarbazone, thifensulfuron(-methyl), thiobencarb, Ti 35, tiocarbazil, topramezone, tralkoxydim, tri-allate, triasulfuron, triaziflam, triazofenamide, tribenuron(-methyl), 2,3,6-trichlorobenzoic acid (2,3,6-TBA), triclopyr, tridiphane, trietazine, trifloxysulfuron(-sodium), trifloxysulfuron, trifluralin, triflusulfuron and esters (e.g. methyl ester, DPX-66037), trimeturon, trinexapac, tritosulfuron, tsitodef, uniconazole, vernolate, WL 110547, i.e. 5-phenoxy-1-[3-(trifluoromethyl)phenyl]-1H-tetrazole, UBH-509, D-489, LS 82-556, KPP-300, NC-324, NC-330, KH-218, DPX-N8189, SC-0774, DOWCO-535, DK-8910, V-53482, PP-600, MBH-001, KIH-9201, ET-751, KIH-6127, KIH-2023, KIH-485 and KIH5996.

Controlling harmful plants selectively is of particular interest in crops of useful plants and ornamentals. Even though the compounds (I) already exhibit very good to sufficient selectivity in many crops, it is possible, in principle, that symptoms of phytotoxicity occur on the cultivated plants in some crops and especially also in the case of mixtures with other herbicides which are less selective. In this respect, combinations of compounds (I) according to the invention which are of particular interest are those which contain the compounds (I) or their combinations with other herbicides or pesticides and safeners. The safeners, which are employed in such an amount that they act as antidote, reduce the phytotoxic side effects of the herbicides/pesticides employed, for example in economically important crops such as cereals (wheat, barley, rye, maize, rice, sorghum and millet), sugar beet, sugar cane, oilseed rape, cotton and soybeans, preferably cereals. The following groups of compounds are examples of suitable safeners for the compounds (I) and their combinations with further pesticides:
a) compounds of the dichlorophenylpyrazoline-3-carboxylic acid type, preferably compounds such as ethyl 1-(2,4-dichlorophenyl)-5- (ethoxycarbonyl)-5-methyl-2-pyrazoline-3-carboxylate (S1-1) ("Mefenpyr-diethyl", PM) and related compounds as they are described in WO 91/07874;
b) dichlorophenylpyrazolecarboxylic acid derivatives, preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S 1-3), ethyl 1-(2,4-dichlorophenyl)-5-(1,1-dimethylethyl)pyrazole-3-carboxylate (S1-4), ethyl 1-(2,4-dichlorophenyl)-5-phenylpyrazole-3-carboxylate (S1-5) and related compounds as they are described in EP-A-333 131 and EP-A-269 806;
c) compounds of the triazolecarboxylic acids type, preferably compounds such as fenchlorazol (and its ethyl ester), i.e. ethyl 1-(2,4-dichlorophenyl)-5-trichloromethyl-(1H)-1,2,4-triazole-3-carboxylate (S1-6), and related compounds (see EP-A-174 562 and EP-A-346 620);
d) compounds of the 5-benzyl- or 5-phenyl-2-isoxazoline-3-carboxylic acid type or the 5,5-diphenyl-2-isoxazoline-3-carboxylic acid, preferably compounds such as ethyl 5-(2,4-dichlorobenzyl)-2-isoxazoline-3-carboxylate (S1-7) or ethyl 5-phenyl-2-isoxazoline-3-carboxylate (S1-8) and related compounds as they are described in WO 91/08202, or ethyl 5,5-diphenyl-2-isoxazolinecarboxylate (S1-9) ("isoxadifen-ethyl") or n-propyl 5,5-diphenyl-2-isoxazolinecarboxylate (S1-10) or ethyl 5-(4-fluorophenyl)-5-phenyl-2-isoxazoline-3-carboxylate (S1-11), as they are described in the international patent application published under WO-A-95/07897;
e) compounds of the 8-quinolinoxyacetic acid type (S2), preferably 1-methylhex-1-yl (5-chloro-8-quinolinoxy)acetate (common name "cloquintocet-mexyl") (S2-1) (see PM) 1,3-dimethylbut-1-yl (5-chloro-8-quinolinoxy)acetate (S2-2); 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl (5-chloro-8-quinolinoxy)acetate (S2-6), allyl (5-chloro-8-quinolinoxy)acetate (S2-7), 2-(2-propylideneiminooxy)-1-ethyl (5-chloro-8-quinolinoxy)acetate (S2-8), 2-oxoprop-1-yl (5-chloro-8-quinolinoxy)acetate (S2-9), and related compounds as are described in EP-A-86 750, EP-A-94 349 and EP-A-191 736 or EP-A-0 492 366;
f) compounds of the (5-chloro-8-quinolinoxy)malonic acid type, preferably compounds such as diethyl (5-chloro-8-quinolinoxy)malonate, diallyl (5-chloro-8-quinolinoxy)malonate, methylethyl (5-chloro-8-quinolinoxy)malonate and related compounds as are described in EP-A-0 582 198;
g) active substances of the phenoxyacetic or phenoxypropionic acid derivatives type or of the aromatic carboxylic acids type, such as, for example, 2,4-dichlorophenoxyacetic acids (and its esters) (2,4-D), 4-chloro-2-methylphenoxypropionic esters (mecoprop), MCPA or 3,6-dichloro-2-methoxybenzoic acid (and its esters) (dicamba);
h) active substances of the pyrimidines type which are employed in rice as soil-acting safeners, such as, for example, "fenclorim" (PM) (= 4,6-dichloro-2-phenylpyrimidine), which is also known as safener for pretilachlor in seeded rice;
i) active substances of the dichloroacetamides type, which are frequently employed as pre-emergence safeners (soil-acting safeners), such as, for example, "dichlormid" (PM) (= N,N-diallyl-2,2-dichloroacetamide), "R-29148" (= 3-dichloroacetyl-2,2,5-trimethyl-1,3-oxazolidine, by Stauffer), "benoxacor" (PM) (= 4-dichloroacetyl-3,4-dihydro-3-methyl-2H-1,4-benzoxazine), "PPG-1292" (= N-allyl-N-[(1,3-dioxolan-2-yl)methyl]dichloroacetamide by PPG Industries), "DK-24" (= N-allyl-N-[(allylaminocarbonyl)methyl]dichloroacetamide by Sagro-Chem), "AD-67" or "MON 4660" (= 3-dichloroacetyl-1-oxa-3-azaspiro[4,5]decane by Nitrokemia and Monsanto, respectively), "diclonon" or "BAS145138" or "LAB145138" (= 3-dichloroacetyl-2,5,5-trimethyl-1,3-diazabicyclo[4.3.0]nonane by BASF) and "furilazol" or "MON 13900" (see PM) (= (RS)-3-dichloroacetyl-5-(2-furyl)-2,2-dimethyloxazolidine);
j) active substances of the dichloroacetone derivatives type, such as, for example,
   "MG 191" (CAS Reg. No. 96420-72-3) (= 2-dichloromethyl-2-methyl-1,3-dioxolane by Nitrokemia), which is known as safener for maize;
k) active substances of the oxyimino compounds type, which are known as seed treatment products, such as, for example,
   "oxabetrinil" (PM) (= (Z)-1,3-dioxolan-2-ylmethoxyimino(phenyl)acetonitrile), which is known as seed-treatment safener for sorghum and millet against metolachlor damage,
   "fluxofenim" (PM) (= 1-(4-chlorophenyl)-2,2,2-trifluoro-1-ethanone O-(1,3-dioxolan-2-ylmethyl)oxime, which is known as seed-dressing safener for sorghum and millet against metolachlor damage, and "cyometrinil" or "-CGA-43089" (PM) (= (Z)-cyanomethoxyimino(phenyl)acetonitrile), which is known as seed-treatment safener for
   sorghum and millet against metolachlor damage;
l) active substances of the thiazolecarboxylic ester type, which are known as seed treatment products, such as, for example,
   "flurazol" (PM) (= benzyl 2-chloro-4-trifluoromethyl-1,3-thiazole-5-carboxylate), which is known as seed-treatment safener for sorghum and
   millet against alachlor and metolachlor damage;
m) active substances of the naphthalenedicarboxylic acid derivatives type, which are known as seed treatment products, such as, for example,
   "naphthalic anhydride" (PM) (= 1,8-naphthalenedicarboxylic anhydride), which is known as seed-treatment safener for maize against thiocarbamate
   herbicide damage;
n) active substances of the chromanacetic acid derivatives type, such as, for example,
   "CL 304415" (CAS Reg. No. 31541-57-8) (= 2-(4-carboxychroman-4-yl)acetic acid by American Cyanamid), which is known as safener for maize against damage by imidazolinones;
o) active substances which, in addition to a herbicidal action against harmful plants, also exhibit a safener action in connection with crop plants such as rice, such as, for example,
   "dimepiperate" or "MY-93" (PM) (= S-1-methyl-1-phenylethyl piperidine-1-carbothioate), which is known as safener for rice against damage by the herbicide molinate,
   "daimuron" or "SK 23" (PM) (= 1-(1-methyl-1-phenylethyl)-3-p-tolylurea), which is known as safener for rice against damage by the herbicide imazosulfuron,
   "cumyluron" = "JC-940" (= 3-(2-chlorophenylmethyl)-1-(1-methyl-1-phenylethyl)urea, see JP-A-60087254), which is known as safener for rice against damage by several herbicides,
   "methoxyphenone" or "NK 049" (= 3,3'-dimethyl-4-methoxybenzophenone), which is known as safener for rice against damage by several herbicides, "CSB" (= 1-bromo-4-(chloromethylsulfonyl)benzene) (CAS Reg. No. 54091-06-4, by Kumiai), which is known as safener in rice against damage by several herbicides;
p) N-acylsulfonamides of the formula (S3) and their salts 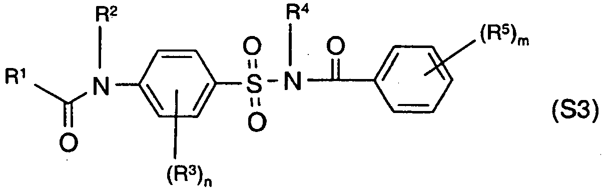 as are described in WO-A-97/45016;
q) acylsulfamoylbenzamides of the formula (S4), if appropriate also in salt form, 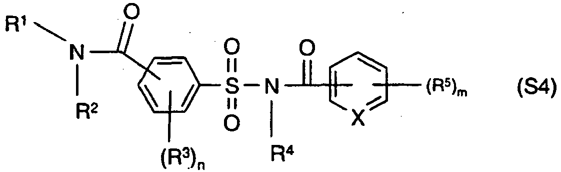 as are described in International Application No. PCT/EP98/06097; and
r) compounds of the formula (S5), 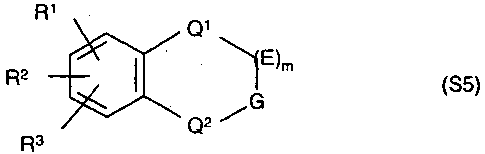 as are described in WO-A 98/13 361,
   including the stereoisomers and the salts conventionally used in agriculture.

Amongst the safeners mentioned, those which are of particular interest are (S1-1) and (S1-9) and (S2-1), in particular (S1-1) and (S1-9).

Some of the safeners are already known as herbicides and therefore simultaneously also display a protective action in connection with the crop plants in addition to the herbicidal action in connection with harmful plants.

The weight ratio of herbicide (mixture) to safener generally depends on the application rate of herbicide and the efficacy of the safener in question; it can vary within wide limits, for example in the range of from 200:1 to 1:200, preferably from 100:1 to 1:100, in particular 20:1 to 1:20. The safeners can be formulated with further herbicides/pesticides, analogously to the compounds (I) or their mixtures, and provided and used as readymix or tank mix together with the herbicides.

For use, the herbicide or herbicide safener formulations, which are present in a customary commercial form, are, if appropriate, diluted in the customary fashion, for example using water in the case of wettable powders, emulsifiable concentrates, dispersions and water-dispersible granules. Preparations in the form of dusts, soil granules, granules for spreading, and sprayable solutions, are usually not diluted further with other inert materials prior to use.

The application rate required of the compounds of the formula (I) varies with, inter alia, the external conditions such as temperature, humidity and the type of the herbicide used. It can vary within wide limits, for example between 0.001 and 10.0 kg/ha or more of active substance, but it is preferably between 0.002 and 3 kg/ha, in particular 0.005 and 1 kg/ha.

### B. Formulation examples

a) A dust is obtained by mixing 10 parts by weight of a compound of formula (I) and 90 parts by weight of talc as inert material and grinding the mixture in a hammer mill.
b) A wettable powder which is readily dispersible in water is obtained by mixing 25 parts by weight of a compound of formula (I), 64 parts by weight of kaolin-containing quartz as inert material, 10 parts by weight of potassium lignosulfonate and 1 part by weight of sodium oleoylmethyltaurinate as wetter and dispersant and grinding the mixture in a pinned-disk mill.
c) A dispersion concentrate which is readily dispersible in water is obtained by mixing 20 parts by weight of a compound of formula (I) with 6 parts by weight of alkylphenol polyglycol ether (®Triton X 207), 3 parts by weight of isotridecanol polyglycol ether (8 EO) and 71 parts by weight of paraffinic mineral oil (boiling range for example approx. 255 to above 277°C) and grinding the mixture in a ball mill to a fineness of below 5 microns.
d) An emulsifiable concentrate is obtained from 15 parts by weight of a compound of formula (I), 75 parts by weight of cyclohexanone as solvent and 10 parts by weight of oxethylated nonylphenol as emulsifier.
e) Water-dispersible granules are obtained by mixing
   75 parts by weight of a compound of formula (I),
   10 parts by weight of calcium ligno-sulfonate,
   5 parts by weight of sodium laurylsulfate,
   3 parts by weight of polyvinyl alcohol and
   7 parts by weight of kaolin,
   grinding the mixture in a pinned disk mill and granulating the powder in a fluidized bed by spraying on water as granulation liquid.
f) Alternatively, water-dispersible granules are obtained by homogenizing and precomminuting, on a colloid mill,
   25 parts by weight of a compound of formula (I),
   5 parts by weight of sodium 2,2'-dinaphthylmethane-6,6'-disulfonate,
   2 parts by weight of sodium oleoylmethyltaurinate,
   1 part by weight of polyvinyl alcohol,
   17 parts by weight of calcium carbonate and
   50 parts by weight of water,
   subsequently grinding the mixture on a bead mill and atomizing and drying the resulting suspension in a spray tower by means of a single-substance nozzle.

### C. Biological examples

### Biological Example 1: Pre-emergence effect on weeds

Seeds or rhizome pieces of monocotyledonous and dicotyledonous weed plants were placed in sandy loam in plastic pots and covered with soil. The compounds according to the invention, which were formulated in the form of wettable powders or emulsion concentrates, were then applied to the surface of the soil cover as aqueous suspension or emulsion at various dosages with an application rate of 600 to 800 I of water per ha (converted).

After the treatment, the pots were placed into a greenhouse and kept under good growth conditions for the weeds. The plant or emergence damage was scored visually after the test plants have emerged after an experimental time of 3 to 4 weeks by comparison with untreated controls.

Compound nos. 1.5, 1.205, 2.1, 2.2, 2.3, 2.5, 2.206, 3.1, 3.31, 3.82, 3.106, 3.109 and 3.112 according to tables 1 to 4 above show a very good pre-emergence control of harmful plants such as Stellaria media, Lolium multiforum, Amaranthus retroflexus, Sinapis alba, Avena sativa and Setaria viridis when applied at an application rate of 0.5 kg or less of active ingredient per hectare. Biological Example 2: Post-emergence effect on weeds

Seeds or rhizome pieces of monocotyledonous and dicotyledonous weeds were placed in sandy loam in plastic pots, covered with soil and grown in the greenhouse under good growth conditions. Three weeks after sowing, the test plants were treated in the three-leaf stage. Various dosages of the compounds according to the invention, which were formulated as wettable powders or emulsion concentrates, were sprayed to the green plant parts at an application rate of 600 to 800 I of water per ha (converted). After the test plants have been left to stand in the greenhouse for approx. 3 to 4 weeks under optimal growth conditions, the effect of the preparations was scored visually by comparison with untreated controls. Compound nos. 1.5, 1.205, 2.1, 2.2, 2.3, 2.5, 2.206, 3.1, 3.31, 3.82, 3.106, 3.109 and 3.112 according to tables 1 to 4 above according to the invention show a very good herbicidal post-emergence activity against against harmful plants such as Sinapis alba, Echinochloa crus-galli, Lolium multiflorum, Stellaria media, Cyperus iria, Amaranthus retroflexus, Setaria viridis, Avena sativa, Lamium purpureum, Matricaria inodora, Papaver rhoeas, Veronica persica, Viola trocolor, Kochia spp and Chenopodium album when applied at an application rate of 2 kg or less of active ingredient per hectare.

### Biological Example 3: Weed control in plantation crops

In further field trials plantation crops were grown under conditions of natural weed infestation and sprayed with various dosages of the substances of formula (I) according to the invention. At various time intervals after the application it is found by means of visual assessment that the compounds according to the invention, such as nos. 1.5, 1.205, 2.1, 2.2, 2.3, 2.5, 2.206, 3.1, 3.31, 3.82, 3.106, 3.109 and 3.112 according to tables 1 to 4 above, leave the plantation crops such as, for example, oil palm, coconut palm, India-rubber tree, citrus, pineapples, cotton, coffee, cocoa and vines unharmed, even at high rates of active substance. The test compounds of formula (I) often show an improved degree of selectivity compared to the prior art compounds, and are therefore suitable for controlling undesired vegetation in plantation crops.

## Claims

1. Compounds of the formula (I) or salts thereof, 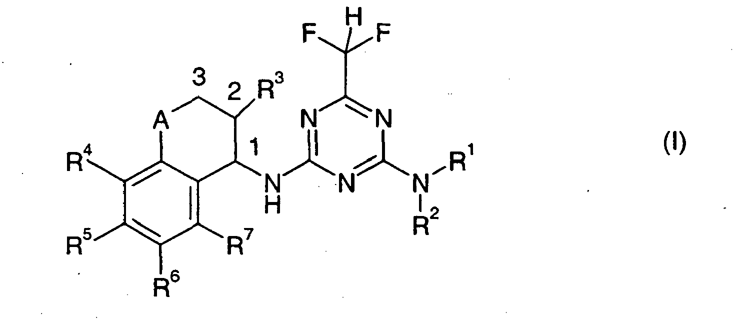 in which
R¹ and R² are each independently H, (C₁-C₄)alkyl, (C₁-C₄)haloalkyl, (C₃-C₄)alkenyl, (C₃-C₄)haloalkenyl, (C₃-C₄)alkynyl, (C₃-C₄)haloalkynyl or an acyl radical,
R³ is H, (C₁-C₆)alkyl, (C₁-C₆)alkoxy, (C₂-C₆)alkenyl, (C₂-C₆)alkinyl, (C₂-C₆)alkenyloxy or (C₂-C₆)alkinyloxy, preferably H, (C₁-C₆)alkneyl or (C₁-C₆)alkoxy,
R⁴, R⁵, R⁶ and R⁷ are each independently H, (C₁-C₄)alkyl, (C₁-C₃)haloalkyl, halogen, (C₁-C₃)alkoxy, (C₁-C₃)haloalkoxy, CN, (C₂-C₆)alkenyl, (C₂-C₆)alkinyl, (C₂-C₆)alkenyloxy or (C₂-C₆)alkinyloxy, but at least one of them is (C₁-C₄)alkyl, (C₁-C₃)haloalkyl, halogen, (C₁-C₃)alkoxy, (C₁-C₃)haloalkoxy, CN, (C₂-C₆)alkenyl, (C₂-C₆)alkinyl, (C₂-C₆)alkenyloxy or (C₂-C₆)alkinyloxy, and
A is CH₂, O or a direct bond.

2. Compounds as claimed in claim 1, **characterized in that**
R¹ is H, (C₁-C₄)alkyl, (C₁-C₄)haloalkyl, (C₃-C₄)alkenyl, (C₃-C₄)alkynyl or an acyl radical having 1 to 12 carbon atoms,
R² is H, (C₁-C₄)alkyl or (C₁-C₄)haloalkyl,
R³ is H, (C₁-C₃)alkyl or (C₁-C₃)alkoxy,
R⁴, R⁵, R⁶ and R⁷ are each independently H, (C₁-C₃)alkyl, halogen or (C₁-C₃)alkoxy and at least one of these is (C₁-C₃)alkyl, halogen or (C₁-C₃)alkoxy, and
A is CH₂ or a direct bond.

3. Compounds as claimed in claim 1 or 2, **characterized in that**
R¹ H, (C₁-C₃)alkyl, (C₁-C₃)haloalkyl, allyl, propargyl, CHO, -CO(C₁-C₃)alkyl or -CO(C₁-C₃)haloalkyl,
R² is H, (C₁-C₂)alkyl,
R³ is H, methyl or ethyl,
R⁴, R⁵ and R⁷ are each independently H, methyl, F, Cl or Br,
R⁶ is methyl, F, Cl or Br, and
A is CH₂ or a direct bond.

4. Compounds as claimed in any of claims 1 to 3, **characterized in that** they are compounds of the formula (I) selected from compounds of formula (Ia) or salts thereof, 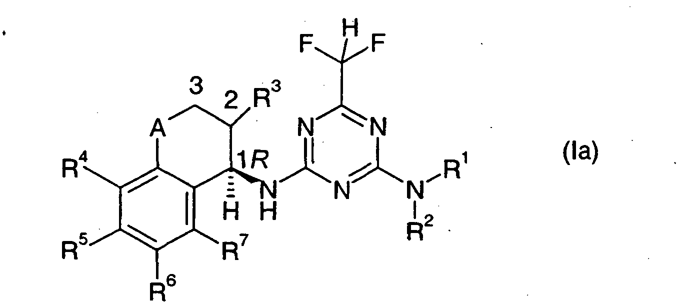 in which the stereochemical configuration at the marked 1 position is 60 to 100 % (R), based on the content of stereoisomers having (R)- and (S)-configurations at this position, and in which R¹, R², R³, R⁴, R⁵, R⁶, R⁷ and A are as defined in formula (I).

5. Compounds as claimed in claim 4, **characterized in that** they are compounds of the formula (I) selected from compounds of the formula (Ib) or salts thereof, 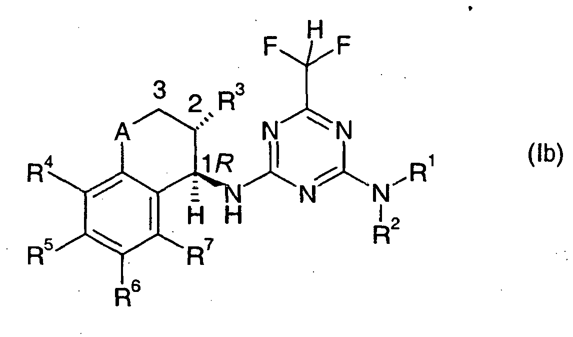 in which R¹, R², R³, R⁴, R⁵, R⁶, R⁷ and A are as defined in formula (I), provided that the R³ group is different from hydrogen and oriented trans to the amino group, and in which the stereochemical configuration at the marked 1 position is 60 to 100 % (*R*), based on the content of stereoisomers having (*R*)- and (*S*)-configurations at this position.

6. Compounds as claimed in claim 4, **characterized in that** they are compounds of the formula (I) selected from compounds of the formula (Ic) or salts thereof, 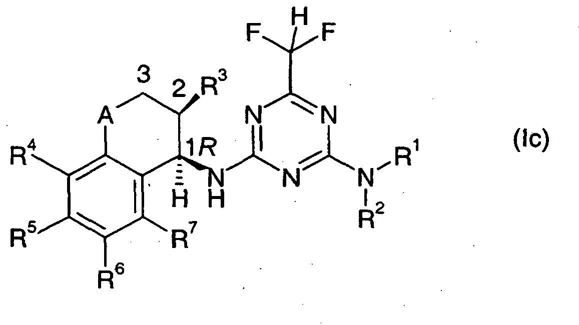 in which R¹, R², R³, R⁴, R⁵, R⁶, R⁷ and A are as defined in formula (I), provided that the R³ group is different from hydrogen and oriented cis to the amino group, and in which the stereochemical configuration at the marked 1 position is 60 to 100 % (*R*), based on the content of stereoisomers having (*R*)- and (*S*)-configurations at this position.

7. Compounds as claimed in any of claims 4 to 6, **characterized in that** R¹ and R² are each H,
R³ is methyl or ethyl,
R⁴, R⁵ and R⁷ are each independently H, methyl, Br, Cl or F, and
R⁶ is methyl, Br, Cl or F, and
A is CH₂, O or a direct bond.

8. Process for the preparation of a compound of formula (I) or a salt thereof as defined in claim 1, **characterized in that**
a) a compound of general formula (II), 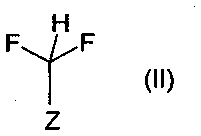 wherein Z is a functional group selected from the group consisting of carboxylic ester, carboxylic orthoester, carboxylic acid chloride, carboxamide, cyano, carboxylic anhydride or trichloromethyl,
is reacted with a biguanidine compound of formula (III) or an acid addition salt thereof, 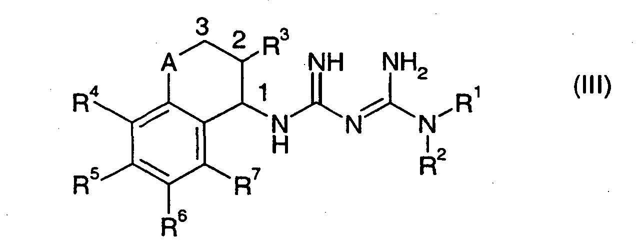 wherein R¹, R², R³, R⁴, R⁵, R⁶, R⁷ and A and the configuration at the marked 1 position are as defined in the compound of formula (I),
to give the compound of formula (I) or a salt thereof, or
b), where one or each of the groups R¹ or R² in the compound of formula (I) to be prepared is (C₁-C₄)alkyl, (C₁-C₄)haloalkyl, (C₃-C₄)alkenyl, (C₃-C₄)haloalkenyl, (C₃-C₄)alkynyl or (C₃-C₄)haloalkynyl,
a compound of formula (I') 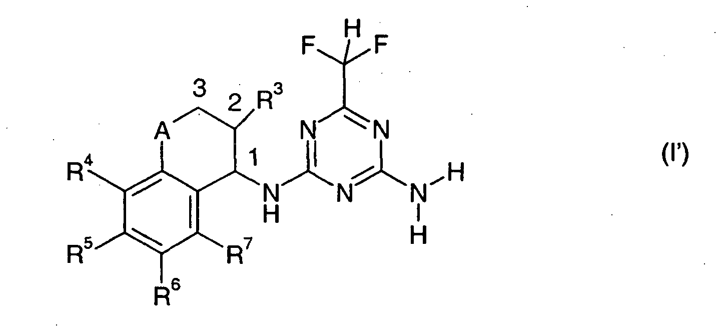 is reacted with an alkylating agent of formula (VI) or (VII) respectively:
R¹-L² (VI)
R²-L² (VII)
wherein L² is a leaving group, to give the compound of the formula (I) or a salt thereof as product of a mono- or dialkylation,
c), where one or each of the groups R¹ or R² in the compound of formula (I) to be prepared is an acyl group, by reacting a compound of formula (I') with an acylating agent of formula (VIII) or (IX) respectively:
R²-L³ (IX)
R¹-L³ (VIII)
to give the compound of the formula (I) or a salt thereof hereof as product of a mono- or diacylation, or
d), where one of the groups R¹ or R² in the compound of formula (I) to be prepared is (C₁-C₄)alkyl, (C₁-C₄)haloalkyl, (C₃-C₄)alkenyl, (C₃-C₄)haloalkenyl, (C₃-C₄)alkynyl or (C₃-C₄)haloalkynyl, and the other is an acyl group, reacting a compound of formula (I') with an alkylating agent of formula (VI) or (VII) and subsequently with an acylating agent of formula (VIII) or (IX), respectively, to give the compound of the formula (I) or a salt thereof, or e) a compound of formula (IV), 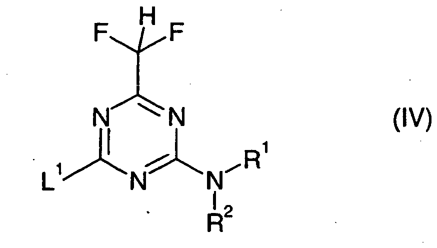 in which R¹ and R² are as defined in the compound of formula (I) to be prepared and L¹ is a leaving group, is reacted with an amine of formula (V), or an acid addition salt thereof, 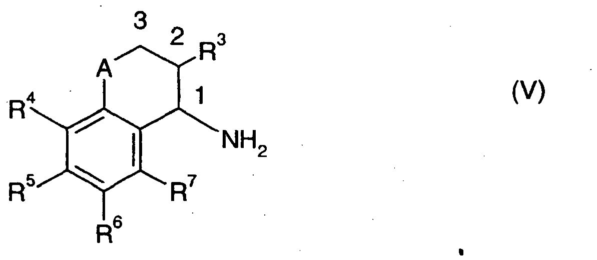 in which R³, R⁴, R⁵, R⁶, R⁷ and A are as defined in the compound of formula (I) to be prepared, or
f) a compound of the formula (IV) 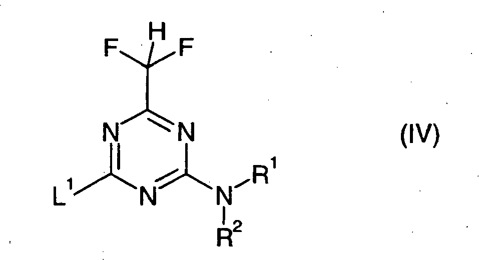 in which R¹ and R² are as defined in the compound of formula (I) to be prepared and L¹ is NH₂, is reacted with a compound of the formula (XII), 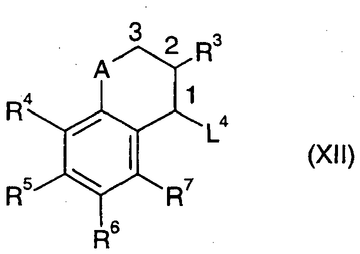 wherein R², R³, R⁴, R⁵, R⁶, R⁷ and A are as defined in the compound of formula (I) to be prepared and L⁴ is a leaving group.

9. A herbicidal or plant growth regulating composition, which comprises one or more compounds of the formula (I) or their salts as claimed in any of claims 1 to 7 and formulation auxiliaries applicable in crop protection.

10. A method of controlling harmful plants or for regulating the growth of plants, which comprises applying an effective amount of one or more compounds of the formula (I) or their salts as claimed in any of claims 1 to 7 at a plant locus.

11. The use of compounds of the formula (I) or their salts as claimed in any of claims 1 to 7 as herbicides or plant growth regulators.

## Patentansprüche

1. Verbindungen der Formel (I) oder Salze davon, 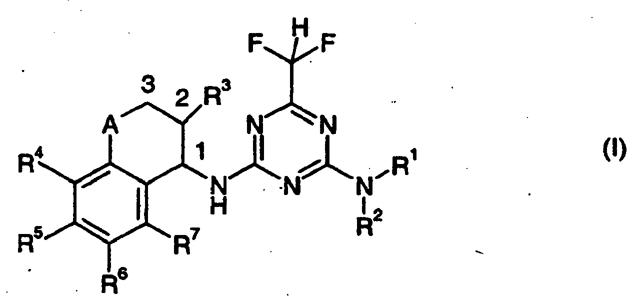 worin
R¹ und R² jeweils unabhängig H, (C₁-C₄)-Alkyl, (C₁-C₄)-Halogenalkyl, (C₃-C₄)-Alkenyl, (C₃-C₄)-Halogenalkenyl, (C₃-C₄)-Alkinyl, (C₃-C₄)-Halogenalkinyl oder einen Acylrest bedeuten,
R³ H, (C₁-C₆) -Alkyl, (C₁-C₆) -Alkoxy, (C₂-C₆) -Alkenyl, (C₂-C₆)-Alkinyl, (C₂-C₆) -Alkenyloxy oder (C₂-C₆)-Alkinyloxy, vorzugweise H, (C₁-C₆)-Alkyl oder (C₁-C₆)-Alkoxy bedeutet,
R⁴, R⁵, R⁶ und R⁷ jeweils unabhängig H, (C₁-C₄)-Alkyl, (C₁-C₃)-Halogenalkyl, Halogen, (C₁-C₃)-Alkoxy, (C₁-C₃)-Halogenalkoxy, CN, (C₂-C₆)-Alkenyl, (C₂-C₆)-Alkinyl, (C₂-C₆)-Alkenyloxy oder (C₂-C₆)-Alkinyloxy bedeuten, wobei jedoch mindestens eines davon (C₁-C₄)-Alkyl, (C₁-C₃) Halogenalkyl, Halogen, (C₁-C₃)-Alkoxy, (C₁-C₃) -Halogenalkoxy, CN, (C₂-C₆)-Alkenyl, (C₂-C₆)-Alkinyl, (C₂-C₆) -Alkenyloxy oder (C₂-C₆)-Alkinyloxy bedeutet, und
A CH₂, 0 oder eine direkte Bindung bedeutet.

2. Verbindungen nach Anspruch 1, **dadurch**
**gekennzeichnet, dass**
R¹ H, (C₁-C₄) -Alkyl, (C₁-C₄) -Halogenalkyl, (C₃-C₄)-Alkenyl, (C₃-C₄)-Alkinyl oder einen Acylrest mit 1 bis 12 Kohlenstoffatomen bedeutet,
R² H, (C₁-C₄)-Alkyl oder (C₁-C₄) -Halogenalkyl bedeutet,
R³ H, (C₁-C₃)-Alkyl oder (C₁-C₃)-Alkoxy bedeutet,
R⁴, R⁵, R⁶ und R⁷ jeweils unabhängig H, (C₁-C₃)-Alkyl, Halogen oder (C₁-C₃)-Alkoxy bedeuten, wobei mindestens eines davon (C₁-C₃)-Alkyl, Halogen oder (C₁-C₃)-Alkoxy bedeutet, und
A CH₂ oder eine direkte Bindung bedeutet.

3. Verbindungen nach Anspruch 1 oder 2, **dadurch**
**gekennzeichnet, dass**
R¹ H, (C₁-C₃)-Alkyl, (C₁-C₃)-Halogenalkyl, Allyl, Propargyl, CHO, -CO(C₁-C₃-Alkyl oder -CO(C₁-C₃)-Halogenalkyl bedeutet,
R² H, (C₁-C₂)-Alkyl bedeutet,
R³ H, Methyl oder Ethyl bedeutet,
R⁴, R⁵ und R⁷ jeweils unabhängig H, Methyl, F, Cl oder Br bedeuten,
R⁶ Methyl, F, Cl oder Br bedeutet, und
A CH₂ oder eine direkte Bindung bedeutet.

4. Verbindungen nach einem der Ansprüche 1 bis 3, **dadurch gekennzeichnet, dass** es sich um Verbindungen der Formel (I) handelt, die aus Verbindungen der Formel (Ia) oder Salzen davon ausgewählt sind, 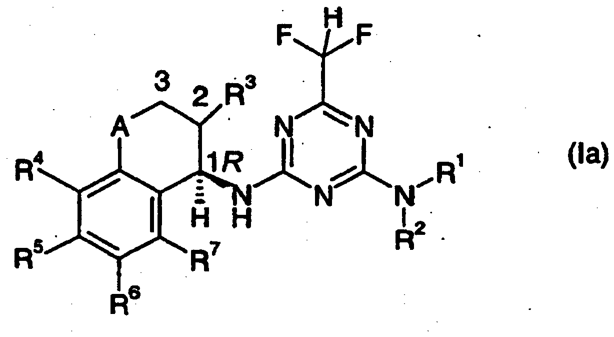 worin die stereochemische Konfiguration an der mit 1 markierten Position 60-100% (*R*), bezogen auf den Gehalt an Stereoisomeren mit (*R*)- und (*S*)-Konfiguration an dieser Position, bedeutet und worin R¹, R², R³, R⁴, R⁵, R⁶, R⁷ und A wie in Formel (I) definiert sind.

5. Verbindungen nach Anspruch 4, **dadurch gekennzeichnet, dass** es sich um Verbindungen der Formel (I) handelt, die aus Verbindungen der Formel (Ib) oder Salzen davon ausgewählt sind, 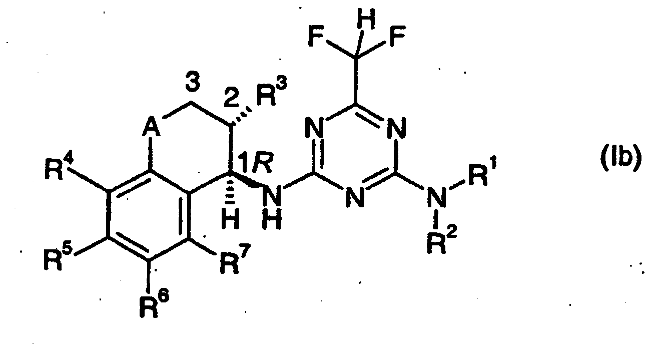 worin R¹, R², R³, R⁴, R⁵, R⁶, R⁷ und A wie in Formel (I) definiert sind, mit der Maßgabe, dass sich die R³-Gruppe von Wasserstoff unterscheidet und in Bezug auf die Aminogruppe in trans-Orientierung vorliegt, und worin die stereochemische Konfiguration an der mit 1 markierten Position 60-100% (*R*), bezogen auf den Gehalt an Stereoisomeren mit (*R*)- und (*S*)-Konfiguration an dieser Position, bedeutet.

6. Verbindungen nach Anspruch 4, **dadurch gekennzeichnet, dass** es sich um Verbindungen der Formel (I) handelt, die aus Verbindungen der Formel (Ic) oder Salzen davon ausgewählt sind, 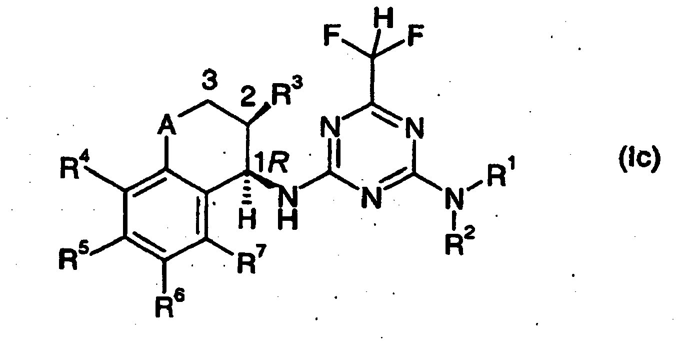 worin R¹, R², R³, R⁴, R⁵, R⁶, R⁷ und A wie in Formel (I) definiert sind, mit der Maßgabe, dass sich die R³-Gruppe von Wasserstoff unterscheidet und in Bezug auf die Aminogruppe in cis-Orientierung vorliegt, und worin die stereochemische Konfiguration an der mit 1 markierten Position 60-100% (*R*), bezogen auf den Gehalt an Stereoisomeren mit (*R*)- und (*S*)-Konfiguration an dieser Position, bedeutet.

7. Verbindungen nach einem der Ansprüche 4 bis 6, **dadurch gekennzeichnet, dass** R¹ und R² jeweils H bedeuten,
R³ Methyl oder Ethyl bedeutet,
R⁴, R⁵ und R⁷ jeweils unabhängig H, Methyl, Br, Cl oder F bedeutet; und
R⁶ Methyl, Br, Cl oder F bedeutet, und
A CH₂, 0 oder eine direkte Bindung bedeutet.

8. Verfahren zur Herstellung einer Verbindung der Formel (I) oder eines Salzes davon wie in Anspruch 1 definiert, **dadurch gekennzeichnet, dass** man
a) eine Verbindung der allgemeinen Formel (II) 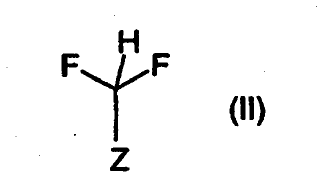 worin Z eine funktionelle Gruppe, ausgewählt aus der Gruppe bestehend aus Carbonsäureester, Carbonsäureorthoester, Carbonsäurechlorid, Carbonsäureamid, Cyano, Carbonsäureanhydrid oder Trichlormethyl bedeutet,
mit einer Biguanidinverbindung der Formel (III) oder einem Säureadditionssalz davon, 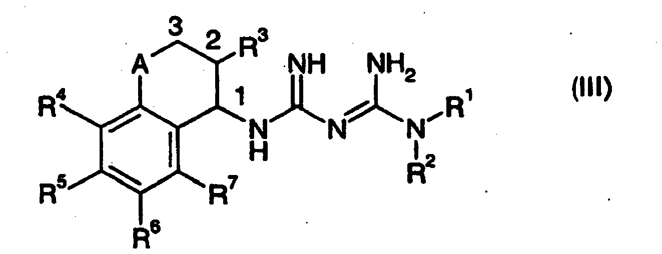 worin R¹, R², R³, R⁴, R⁵, R⁶, R⁷ und A und die Konfiguration an der mit 1 bezeichneten Position wie in der Verbindung von Formel (I) definiert sind,
zu der Verbindung der Formel (I) oder einem Salz davon umsetzt, oder
b) wenn eine oder jede der Gruppen R¹ oder R² in der Verbindung der Formel (I), die es herzustellen gilt, (C₁-C₄-Alkyl, (C₁-C₄) -Halogenalkyl, (C₃-C₄) - Alkenyl, (C₃-C₄) -Halogenalkenyl, (C₃-C₄) -Alkinyl oder (C₃-C₄)-Halogenalkinyl bedeutet, eine Verbindung der Formel (I') 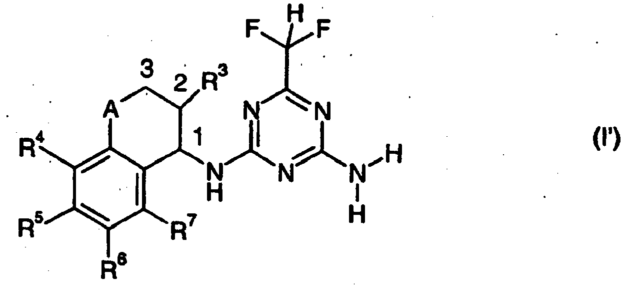 mit einem Alkylierungsmittel der Formel (VI) bzw. (VII) :
R¹-L² (VI)
R²-L² (VII)
worin L² eine Abgangsgruppe bedeutet, zu der Verbindung der Formel (I) oder einem Salz davon als Produkt einer Mono- oder Dialkylierung umsetzt,
c) wenn eine oder jede der Gruppen R¹ oder R² in der Verbindung der Formel (I), die es herzustellen gilt, eine Acylgruppe ist, eine Verbindung der Formel (I') mit einem Acylierungsmittel der Formel (VIII) bzw. (IX):
R¹-L³ (VIII)
R²-L³ (IX)
zu der Verbindung der Formel (I) oder einem Salz davon als Produkt einer Mono- oder Diacylierung umsetzt, oder
d) wenn eine der Gruppen R¹ oder R² in der Verbindung der Formel (I), die es herzustellen gilt, (C₁-C₄)-Alkyl, (C₁-C₄) -Halogenalkyl, (C₃-C₄)-Alkenyl, (C₃-C₄)-Halogenalkenyl, (C₃-C₄)-Alkinyl oder (C₃-C₄)-Halogenalkinyl ist und die andere eine Acylgruppe ist,
eine Verbindung der Formel (I') mit einem Alkylierungsmittel der Formel (VI) oder (VII) und anschließend mit einem Acylierungsmittel der Formel (VIII) bzw. (IX) zu der Verbindung der Formel (I) oder einem Salz davon umsetzt, oder
e) eine Verbindung der Formel (IV), 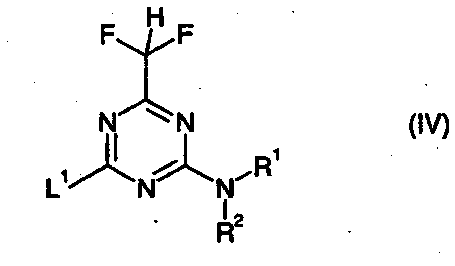 worin R¹ und R² wie in der Verbindung der Formel (I), die es herzustellen gilt, definiert sind und L¹ eine Abgangsgruppe bedeutet,
mit einem Amin der Formel (V) oder einem Säureadditionssalz davon, 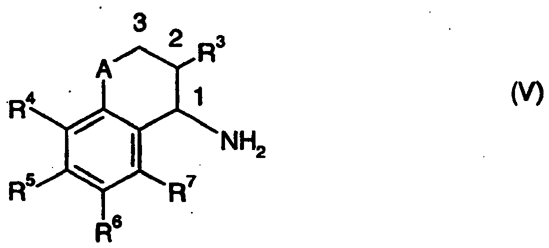 worin R³, R⁴, R⁵, R⁶, R⁷ und A wie in der Verbindung der Formel (I), die es herzustellen gilt, umsetzt,
oder
f) eine Verbindung der Formel (IV) 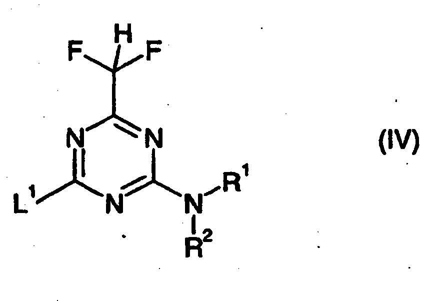 worin R¹ und R² wie in der Verbindung der Formel (I), die es herzustellen gilt, definiert sind und L¹ NH₂ bedeutet, mit einer Verbindung der Formel
(XII), 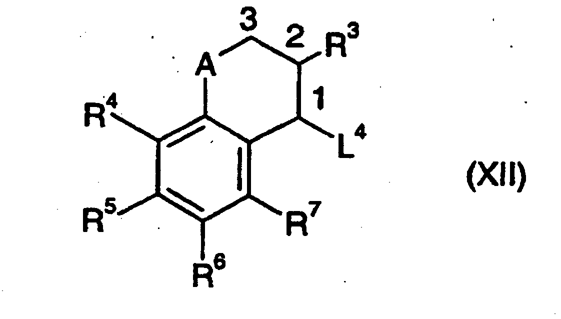 worin R², R³, R⁴, R⁵, R⁶, R⁷ und A wie in der Verbindung der Formel (I), die es herzustellen gilt, definiert sind und L⁴ eine Abgangsgruppe bedeutet, umsetzt.

9. Herbizide oder pflanzenwachstumsregulierende Zusammensetzung, die eine oder mehrere Verbindungen der Formel (I) oder ihre Salze nach einem der Ansprüche 1 bis 7 sowie Formulierungshilfsstoffe, die im Pflanzenschutz einsetzbar sind, umfasst.

10. Verfahren zum Bekämpfen von Schadpflanzen oder zum Regulieren des Wachstums von Pflanzen, bei dem man eine wirksame Menge an einer oder mehreren Verbindungen der Formel (I) oder ihrer Salze nach einem der Ansprüche 1 bis 7 auf einen Pflanzen-Locus ausbringt.

11. Verwendung von Verbindungen der Formel (I) oder ihrer Salze nach einem der Ansprüche 1 bis 7 als Herbizide oder Pflanzenwachtsumsregulatoren.

## Revendications

1. Composés représentés par la formule (I) ou sels de ceux-ci, 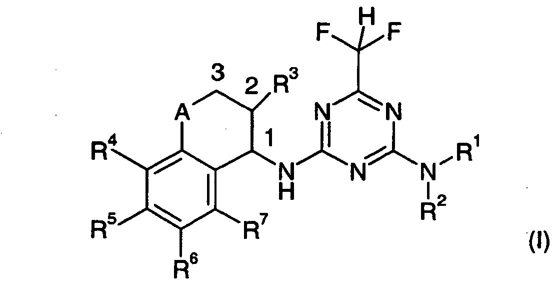 dans lesquels
R¹ et R² sont chacun indépendamment H, un groupe alkyle en C₁-C₄, halogénoalkyle en C₁-C₄, alcényle en C₃-C₄, halogénoalcényle en C₃-C₄, alcynyle en C₃-C₄ ou halogénoalcynyle en C₃-C₄ ou un radical acyle,
R³ est H ou un groupe alkyle en C₁-C₆, alcoxy en C₁-C₆, alcényle en C₂-C₆, alcynyle en C₂-C₆, alcényloxy en C₂-C₆ ou alcynyloxy en C₂-C₆, de préférence H ou un groupe alkyle en C₁-C₆ ou alcoxy en C₁-C₆,
R⁴, R⁵, R⁶ et R⁷ sont chacun indépendamment H ou un groupe alkyle en C₁-C₄, halogénoalkyle en C₁-C₃, halogéno, alcoxy en C₁-C₃, halogénoalcoxy en C₁-C₃, CN, alcényle en C₂-C₆, alcynyle en C₂-C₆, alcényloxy en C₂-C₆ ou alcynyloxy en C₂-C₆, mais au moins l'un d'eux est un groupe alkyle en C₁-C₄, halogénoalkyle en C₁-C₃, halogéno, alcoxy en C₁-C₃, halogénoalcoxy en C₁-C₃, CN, alcényle en C₂-C₆, alcynyle en C₂-C₆, alcényloxy en C₂-C₆ ou alcynyloxy en C₂-C₆, et
A est CH₂, 0 ou une liaison directe.

2. Composés selon la revendication 1, **caractérisés en ce que**
R¹ est H, un groupe alkyle en C₁-C₄, halogénoalkyle en C₁-C₄, alcényle en C₃-C₄ ou alcynyle en C₃-C₄ ou un radical acyle ayant 1 à 12 atomes de carbone,
R² est H ou un groupe alkyle en C₁-C₄ ou halogénoalkyle en C₁-C₄,
R³ est H ou un groupe alkyle en C₁-C₃ ou alcoxy en C₁-C₃,
R⁴, R⁵, R⁶ et R⁷ sont chacun indépendamment H ou un groupe alkyle en C₁-C₃, halogéno ou alcoxy en C₁-C₃ et au moins l'un de ceux-ci est un groupe alkyle en C₁-C₃, halogéno ou alcoxy en C₁-C₃ et
A est CH₂ ou une liaison directe.

3. Composés selon la revendication 1 ou 2, **caractérisés en ce que**
R¹ est H ou un groupe alkyle en C₁-C₃, halogénoalkyle en C₁-C₃, allyle, propargyle, CHO, -CO(alkyle en C₁-C₃) ou -CO(halogénoalkyle en C₁-C₃),
R² est H ou un groupe alkyle en C₁-C₂,
R³ est H ou un groupe méthyle ou éthyle,
R⁴, R⁵ et R⁷ sont chacun indépendamment H, un groupe méthyle, F, Cl ou Br,
R⁶ est un groupe méthyle, F, Cl ou Br et
A est CH₂ ou une liaison directe.

4. Composés selon l'une quelconque des revendications 1 à 3, **caractérisés en ce qu'**ils sont des composés représentés par la formule (I) choisis parmi les composés de formule (Ia) ou les sels de ceux-ci, 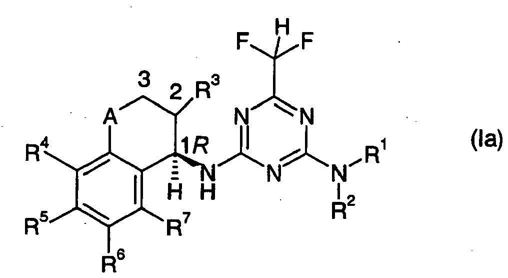 dans lesquels la configuration stéréochimique au niveau de la position marquée 1 est à 60 à 100 % (*R*), sur la base de la teneur des stéréoisomères ayant des configurations (*R*) et (*S*) au niveau de cette position, et dans lesquels R¹, R², R³, R⁴, R⁵, R⁶, R⁷ et A sont tels que définis dans la formule (I).

5. Composés selon la revendication 4, **caractérisés en ce qu'**ils sont des composés représentés par la formule (I) choisis parmi les composés représentés par la formule (Ib) ou les sels de ceux-ci, 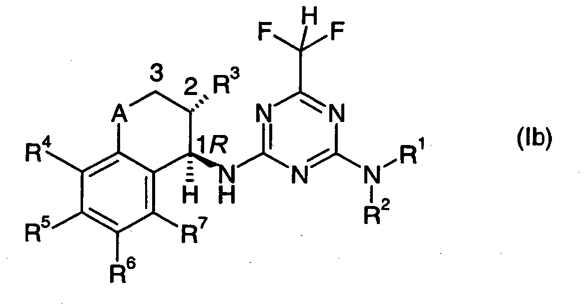 dans lesquels R¹, R², R³, R⁴, R⁵, R⁶, R⁷ et A sont tels que définis dans la formule (I), à condition que le groupe R³ soit différent de l'atome d'hydrogène et orienté en trans par rapport au groupe amino, et dans lesquels la configuration stéréochimique au niveau de la position marquée 1 est à 60 à 100 % (*R*), sur la base de la teneur des stéréoisomères ayant des configurations (*R*) et (*S*) au niveau de cette position.

6. Composés selon la revendication 4, **caractérisés en ce qu'**ils sont des composés représentés par la formule (I) choisis parmi les composés représentés par la formule (Ic) ou les sels de ceux-ci, 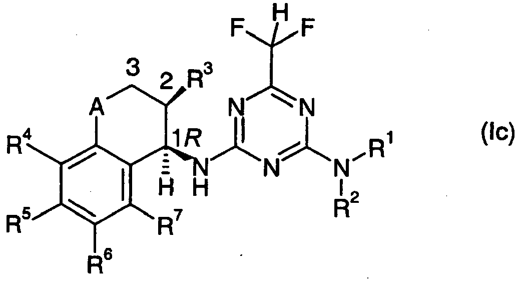 dans lesquels R¹, R², R³, R⁴, R⁵, R⁶, R⁷ et A sont tels que définis dans la formule (I), à condition que le groupe R³ soit différent de l'atome d'hydrogène et orienté en cis par rapport au groupe amino, et dans lesquels la configuration stéréochimique au niveau de la position marquée 1 est à 60 à 100 % (*R*), sur la base de la teneur des stéréoisomères ayant des configurations (*R*) et (*S*) au niveau de cette position.

7. Composés selon l'une quelconque des revendications 4 à 6, **caractérisés en ce que**
R¹ et R² sont chacun H,
R³ est un groupe méthyle ou éthyle,
R⁴, R⁵ et R⁷ sont chacun indépendamment H, un groupe méthyle, Br, Cl ou F et
R⁶ est un groupe méthyle, Br, Cl ou F et
A est CH₂, O ou une liaison directe.

8. Procédé pour la préparation d'un composé de formule (I) ou d'un sel de celui-ci tel que défini dans la revendication 1, **caractérisé en ce que**
a) un composé de formule générale (II), 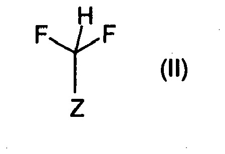 dans lequel Z est un groupe fonctionnel choisi dans le groupe constitué par les groupes ester d'acide carboxylique, orthoester d'acide carboxylique, chlorure d'acide carboxylique, carboxamide, cyano, anhydride carboxylique ou trichlorométhyle,
est amené à réagir avec un composé de biguanidine de formule (III) ou un sel d'addition d'acide de celui-ci, 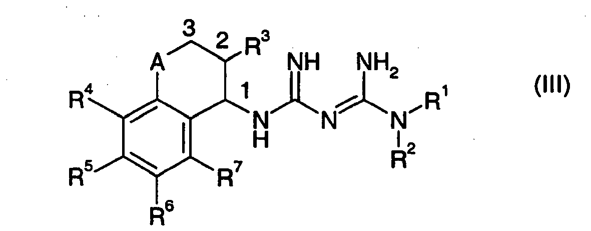 dans lequel R¹, R², R³, R⁴, R⁵, R⁶, R⁷ et A et la configuration au niveau de la position marquée 1 sont tels que définis dans le composé de formule (I),
pour donner le composé de formule (I) ou un sel de celui-ci, ou
b) quand l'un ou chacun des groupes R¹ ou R² dans le composé de formule (I) devant être préparé est un groupe alkyle en C₁-C₄, halogénoalkyle en C₁-C₄,
alcényle en C₃-C₄, halogénoalcényle en C₃-C₄, alcynyle en C₃-C₄ ou halogénoalcynyle en C₃-C₄,
un composé de formule (I') 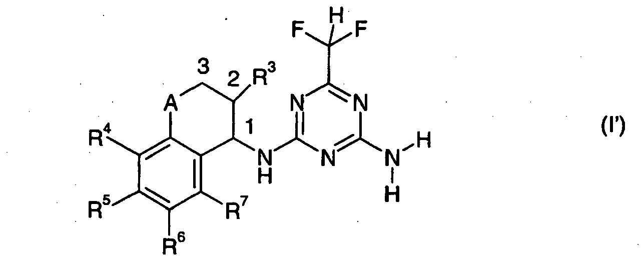 est amené à réagir avec un agent d'alkylation de formule (VI) ou (VII) respectivement :
R¹-L² (VI)
R²-L² (VII)
dans lequel L² est un groupe partant, pour donner le composé représenté par la formule (I) ou un sel de celui-ci en tant que produit d'une monoalkylation ou dialkylation,
c) quand l'un ou chacun des groupes R¹ ou R² dans le composé de formule (I) devant être préparé est un groupe acyle, un composé de formule (I') est amené à réagir avec un agent d'acylation de formule (VIII) ou (IX) respectivement :
R¹-L³ (VIII)
R²-L³ (IX)
pour donner le composé représenté par la formule (I) ou un sel de celui-ci en tant que produit d'une monoacylation ou diacylation, ou
d) quand l'un des groupes R¹ ou R² dans le composé de formule (I) devant être préparé est un groupe alkyle en C₁-C₄, halogénoalkyle en C₁-C₄, alcényle en C₃-C₄, halogénoalcényle en C₃-C₄, alcynyle en C₃-C₄ ou halogénoalcynyle en C₃-C₄, et l'autre est un groupe acyle, un composé de formule (I') est amené à réagir avec un agent d'alkylation de formule (VI) ou (VII) et par la suite avec un agent d'acylation de formule (VIII) ou (IX), respectivement, pour donner le composé représenté par la formule (I) ou un sel de celui-ci, ou
e) un composé de formule (IV), 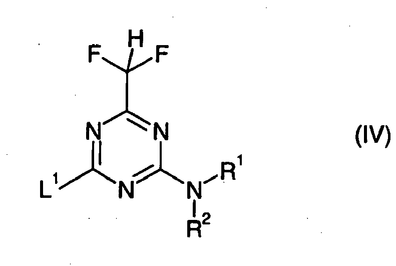 dans lequel R¹ et R² sont tels que définis dans le composé de formule (I) devant être préparé et L¹ est un groupe partant, est amené à réagir avec une amine de formule (V), ou un sel d'addition d'acide de celle-ci, 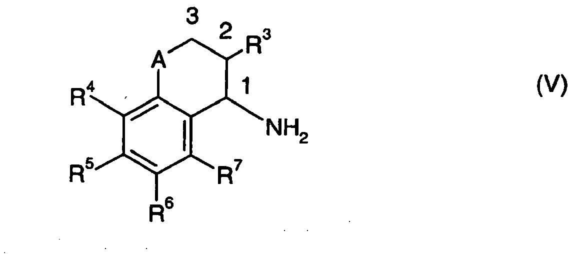 dans laquelle R³, R⁴, R⁵, R⁶, R⁷ et A sont tels que définis dans le composé de formule (I) devant être préparé, ou
f) un composé représenté par la formule (IV) 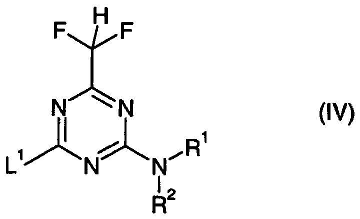 dans lequel R¹ et R² sont tels que définis dans le composé de formule (I) devant être préparé et L¹ est NH₂, est amené à réagir avec un composé représenté par la formule (XII), 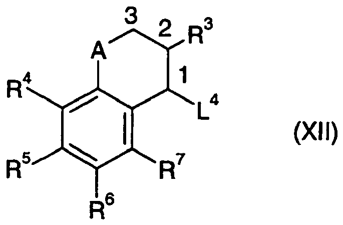
dans lequel R², R³, R⁴, R⁵, R⁶, R⁷ et A sont tels que définis dans le composé de formule (I) devant être préparé et L⁴ est un groupe partant.

9. Composition herbicide ou de régulation de la croissance de plantes, qui comprend un ou plusieurs composés représentés par la formule (I) ou leurs sels selon l'une quelconque des revendications 1 à 7 et des adjuvants de formulation applicables en protection des cultures.

10. Procédé de lutte contre des plantes préjudiciables ou pour la régulation de la croissance de plantes, qui comprend l'application d'une quantité efficace d'un ou plusieurs composés représentés par la formule (I) ou de leurs sels selon l'une quelconque des revendications 1 à 7 sur un lieu de croissance de plantes.

11. Utilisation de composés représentés par la formule (I) ou de leurs sels selon l'une quelconque des revendications 1 à 7 comme herbicides ou régulateurs de la croissance de plantes.
